(19) Europäisches Patentamt
European Patent Office
Office européen des brevets

(11) **EP 3 243 067 B1**

(12) **EUROPEAN PATENT SPECIFICATION**

(45) Date of publication and mention of the grant of the patent:
**08.04.2020 Bulletin 2020/15**

(51) Int Cl.:
***G01N 21/359*** *(2014.01)*    ***G01N 33/28*** *(2006.01)*

(21) Application number: **16706450.0**

(22) Date of filing: **05.01.2016**

(86) International application number:
**PCT/US2016/012171**

(87) International publication number:
**WO 2016/112004 (14.07.2016 Gazette 2016/28)**

(54) **CHARACTERIZATION OF CRUDE OIL BY NEAR INFRARED SPECTROSCOPY**

CHARAKTERISIERUNG VON ROHÖL DURCH NAHINFRAROT-SPEKTROSKOPIE

CARACTÉRISATION DU PÉTROLE BRUT AU MOYEN D'UNE SPECTROSCOPIE PROCHE INFRAROUGE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **05.01.2015 US 201562099704 P**

(43) Date of publication of application:
**15.11.2017 Bulletin 2017/46**

(73) Proprietor: **Saudi Arabian Oil Company Dhahran 31311 (SA)**

(72) Inventors:
• **KOSEOGLU, Omer Refa Dhahran 31311 (SA)**

• **AL-HAJJI, Adnan Dhahran 31311 (SA)**

(74) Representative: **Zaccaro, Elisabetta et al Notarbartolo & Gervasi S.p.A. Corso di Porta Vittoria, 9 20122 Milano (IT)**

(56) References cited:
**EP-A1- 0 984 277    WO-A2-2009/082418**

• **FALLA F S ET AL: "Characterization of crude petroleum by NIR", JOURNAL OF PETROLEUM SCIENCE AND ENGINEERING, ELSEVIER, AMSTERDAM, NL, vol. 51, no. 1-2, 16 April 2006 (2006-04-16), pages 127-137, XP025115453, ISSN: 0920-4105, DOI: 10.1016/J.PETROL.2005.11.014 [retrieved on 2006-04-16]**

EP 3 243 067 B1

**Description**

**Related Applications**

This application claims the benefit of U.S. Provisional Patent Application No. 62/099,704 filed January 5, 2015.

**Field of the Invention**

This invention relates to a method and process for the evaluation of samples of crude oil and its fractions by near infrared spectroscopy.

**Background of the Invention**

Crude oil originates from the decomposition and transformation of aquatic, mainly marine, living organisms and/or land plants that became buried under successive layers of mud and silt some 15-500 million years ago. They are essentially very complex mixtures of many thousands of different hydrocarbons. Depending on the source, the oil predominantly contains various proportions of straight and branched-chain paraffins, cycloparaffins, and naphthenic, aromatic, and polynuclear aromatic hydrocarbons. These hydrocarbons can be gaseous, liquid, or solid under normal conditions of temperature and pressure, depending on the number and arrangement of carbon atoms in the molecules.

Crude oils vary widely in their physical and chemical properties from one geographical region to another and from field to field. Crude oils are usually classified into three groups according to the nature of the hydrocarbons they contain: paraffinic, naphthenic, asphaltic, and their mixtures. The differences are due to the different proportions of the various molecular types and sizes. One crude oil can contain mostly paraffins, another mostly naphthenes. Whether paraffinic or naphthenic, one can contain a large quantity of lighter hydrocarbons and be mobile or contain dissolved gases; another can consist mainly of heavier hydrocarbons and be highly viscous, with little or no dissolved gas. Crude oils can also include heteroatoms containing sulfur, nitrogen, nickel, vanadium and other elements in quantities that impact the refinery processing of the crude oil fractions. Light crude oils or condensates can contain sulfur in concentrations as low as 0.01 W%; in contrast, heavy crude oils can contain as much as 5-6 W%. Similarly, the nitrogen content of crude oils can range from 0.001-1.0 W%.

The nature of the crude oil governs, to a certain extent, the nature of the products that can be manufactured from it and their suitability for special applications. A naphthenic crude oil will be more suitable for the production of asphaltic bitumen, a paraffinic crude oil for wax. A naphthenic crude oil, and even more so an aromatic one, will yield lubricating oils with viscosities that are sensitive to temperature. However, with modern refining methods there is greater flexibility in the use of various crude oils to produce many desired type of products.

A crude oil assay is a traditional method of determining the nature of crude oils for benchmarking purposes. Crude oils are subjected to true boiling point (TBP) distillations and fractionations to provide different boiling point fractions. The crude oil distillations are carried out using the American Standard Testing Association (ASTM) Method D 2892. The common fractions and their nominal boiling points are given in Table 1.

**Table 1**

| Fraction | Boiling Point, °C |
|---|---|
| Methane | -161.5 |
| Ethane | -88.6 |
| Propane | -42.1 |
| Butanes | -6.0 |
| Light Naphtha | 36-90 |
| Mid Naphtha | 90-160 |
| Heavy Naphtha | 160-205 |
| Light Gas Oil | 205-260 |
| Mid Gas Oil | 260-315 |
| Heavy Gas Oil | 315-370 |
| Light Vacuum Gas Oil | 370-430 |

(continued)

| Fraction | Boiling Point, °C |
|---|---|
| Mid Vacuum Gas Oil | 430-480 |
| Heavy Vacuum Gas oil | 480-565 |
| Vacuum Residue | 565+ |

The yields, composition, physical and indicative properties of these crude oil fractions, where applicable, are then determined during the crude assay work-up calculations. Typical compositional and property information obtained from a crude oil assay is given in Table 2.

**Table 2**

| Property | Unit | Property Type | Fraction |
|---|---|---|---|
| Yield Weight and Volume % | W% | Yield | All |
| API Gravity | ° | Physical | All |
| Viscosity Kinematic @ 38 °C | ° | Physical | Fraction boiling >250 °C |
| Refractive Index @ 20 °C | Unitless | Physical | Fraction boiling <400 °C |
| Sulfur | W% | Composition | All |
| Mercaptan Sulfur, W% | W% | Composition | Fraction boiling <250 °C |
| Nickel | Ppmw | Composition | Fraction boiling >400 °C |
| Nitrogen | Ppmw | Composition | All |
| Flash Point, COC | °C | Indicative | All |
| Cloud Point | °C | Indicative | Fraction boiling >250 °C |
| Pour Point, (Upper) | °C | Indicative | Fraction boiling >250 °C |
| Freezing Point | °C | Indicative | Fraction boiling >250 °C |
| Microcarbon Residue | W% | Indicative | Fraction boiling >300 °C |
| Smoke Point, mm | mm | Indicative | Fraction boiling between 150-250 |
| Cetane Index | Unitless | Indicative | Fraction boiling between 150-400 |
| Aniline Point | °C | Indicative | Fraction boiling <520 °C |

Due to the number of distillation cuts and the number of analyses involved, the crude oil assay work-up is both costly and time consuming.

In a typical refinery, crude oil is first fractionated in the atmospheric distillation column to separate sour gas and light hydrocarbons, including methane, ethane, propane, butanes and hydrogen sulfide, naphtha (36°-180°C), kerosene (180°-240°C), gas oil (240°-370°C) and atmospheric residue (>370°C). The atmospheric residue from the atmospheric distillation column is either used as fuel oil or sent to a vacuum distillation unit, depending on the configuration of the refinery. The principal products obtained from vacuum distillation are vacuum gas oil, comprising hydrocarbons boiling in the range 370°-520°C, and vacuum residue, comprising hydrocarbons boiling above 520°C. Crude assay data is conventionally obtained from individual analysis of these cuts to help refiners to understand the general composition of the crude oil fractions and properties so that the fractions can be processed most efficiently and effectively in an appropriate refining unit. Indicative properties are used to determine the engine/fuel performance or usability or flow characteristic or composition. A summary of the indicative properties and their determination methods with description is given below.

Viscosity is a measure of the resistance of a fluid which is being deformed by either shear stress or tensile stress. Viscosity describes a fluid's internal resistance to flow and may be thought of as a measure of fluid friction. All real fluids (except superfluids) have some resistance to stress, but a fluid which has no resistance to shear stress is known as an ideal fluid or inviscid fluid. Viscosity of many petroleum fuels is important for the estimation of process units, optimum storage, handling, and operational conditions and determined by ASTM method D445.

The cetane number of diesel fuel oil, determined by the ASTM D613 method, provides a measure of the ignition quality of diesel fuel; as determined in a standard single cylinder test engine; which measures ignition delay compared to primary reference fuels. The higher the cetane number; the easier the high-speed; direct-injection engine will start; and the less white smoking and diesel knock after start-up are. The cetane number of a diesel fuel oil is determined by comparing its combustion characteristics in a test engine with those for blends of reference fuels of known cetane number under standard operating conditions. This is accomplished using the bracketing hand wheel procedure which varies the compression ratio (hand wheel reading) for the sample and each of the two bracketing reference fuels to obtain a specific ignition delay, thus permitting interpolation of cetane number in terms of hand wheel reading.

The cloud point, determined by the ASTM D2500 method, is the temperature at which a cloud of wax crystals appears when a lubricant or distillate fuel is cooled under standard conditions. Cloud point indicates the tendency of the material to plug filters or small orifices under cold weather conditions. The specimen is cooled at a specified rate and examined periodically. The temperature at which cloud is first observed at the bottom of the test jar is recorded as the cloud point. This test method covers only petroleum products and biodiesel fuels that are transparent in 40 mm thick layers, and with a cloud point below 49°C.

The pour point of petroleum products, determined by the ASTM D97 method, is an indicator of the ability of oil or distillate fuel to flow at cold operating temperatures. It is the lowest temperature at which the fluid will flow when cooled under prescribed conditions. After preliminary heating, the sample is cooled at a specified rate and examined at intervals of 3°C for flow characteristics. The lowest temperature at which movement of the specimen is observed is recorded as the pour point.

The aniline point, determined by the ASTM D611 method, is the lowest temperature at which equal volumes of aniline and hydrocarbon fuel or lubricant base stock are completely miscible. A measure of the aromatic content of a hydrocarbon blend is used to predict the solvency of a base stock or the cetane number of a distillate fuel Specified volumes of aniline and sample, or aniline and sample plus n-heptane, are placed in a tube and mixed mechanically. The mixture is heated at a controlled rate until the two phases become miscible. The mixture is then cooled at a controlled rate and the temperature at which two phases separate is recorded as the aniline point or mixed aniline point.

To determine these properties of gas oil or naphtha fractions conventionally, these fractions have to be distilled off from the crude oil and then measured / determined using various analytical methods that are laborious, costly and time consuming.

Infrared energy is the electromagnetic energy of molecular vibration. The energy band is defined for convenience as the near infrared (0.78-2.50 microns), the infrared (or mid-infrared) 2.50-40.0 microns, and the far infrared (40.0-1000 microns).

"Characterization of crude petroleum by NIR" by F.S. Falla et al. (Journal of Petroleum Science and Engineering 51, 2006) discloses a method for the estimation of simulated distillation properties of crude petroleum based on near infrared spectroscopy.

New rapid and direct methods to help better understand crude oil composition and properties from analysis of whole crude oil will save producers, marketers, refiners and/or other crude oil users substantial expense, effort and time. Therefore, a need exists for an improved system and method for determining indicative properties of crude oil fractions from different sources.

## Summary of the Invention

Systems and methods for assigning one or more distillation temperatures for one or more given distillation weight percentages of a crude oil sample are provided, which can be used to produce a simulated distillation curve. Simulated distillation temperatures of crude oil samples are assigned as a function of density and data derived from direct near infrared spectroscopy measurement of the crude oil samples. The correlations also provide information about the gas oil indicative properties without fractionation/distillation (crude oil assays) and will help producers, refiners, and marketers to benchmark the oil quality and, as a result, valuate the oils without performing the customary extensive and time-consuming crude oil assays.

## Brief Description of the Drawing

Further advantages and features of the present invention will become apparent from the following detailed description of the invention when considered with reference to the accompanying drawings, in which:

FIG. 1 is a graphic plot of typical near infrared spectroscopy data for three types of crude oil;
FIG. 2 is a process flow diagram of steps carried out to characterize distillation data of a crude oil sample, using the system and method herein; and
FIG. 3 is a block diagram of a component of a system for implementing the invention, according to one embodiment.

## Detailed Description of Invention

A system and method is provided for determining distillation data of a hydrocarbon sample. The systems and methods are applicable for naturally occurring hydrocarbons derived from crude oils, bitumens, heavy oils, shale oils and from refinery process units including hydrotreating, hydroprocessing, fluid catalytic cracking, coking, and visbreaking or coal liquefaction. Samples can be obtained from various sources, including an oil well, stabilizer, extractor, or distillation tower.

A method for determining boiling point distribution of a hydrocarbon oil based upon near infrared spectroscopy data derived from a sample of the hydrocarbon oil and the density of the sample is provided. The sample is prepared for near infrared spectroscopy analysis. Spectra data for the sample is obtained by a near infrared spectroscopy analysis. The spectra data obtained by near infrared spectroscopy analysis of the sample is entered into the computer. Cumulative near infrared absorbance of the hydrocarbon oil is calculated from the near infrared spectroscopy data. Cumulative near infrared absorbance of the hydrocarbon oil is normalized to 100 W%. The wavenumber is determined at 0.5, 5, 10, 20, 30, 40, 50, 60, 70, 80, 90, 95, 99.5 W% points. Boiling point distribution of hydrocarbon oil is calculated from normalized near infrared data and the density of hydrocarbon oil. The wavenumber of near infrared spectrum can be in the range 4,000-12,821 cm-1.

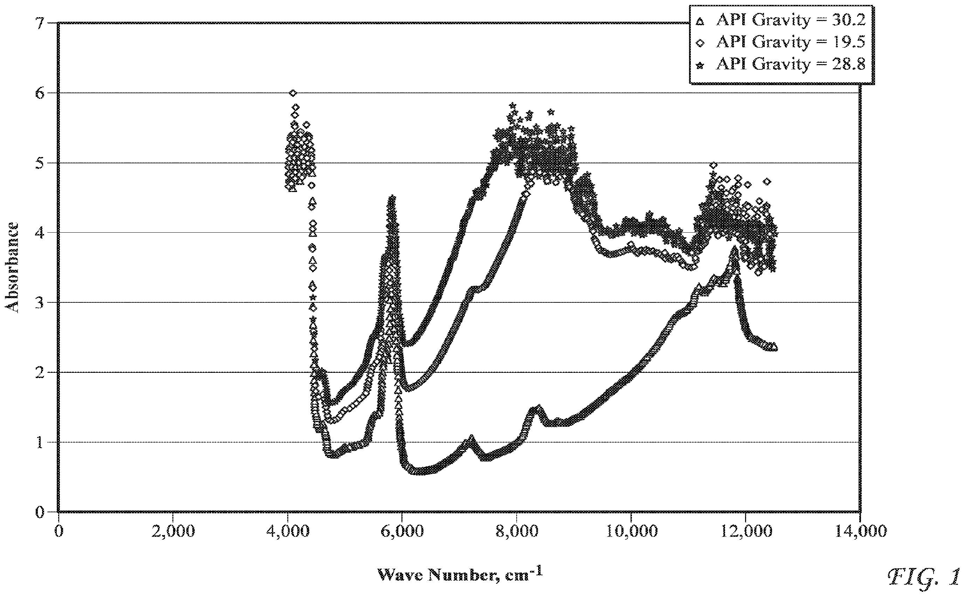

In the system and method herein, spectra are obtained by a suitable known or to be developed near infrared spectroscopy techniques, for instance, to obtain graphic plots of near infrared spectroscopy data as shown in FIG. 1. Infrared energy is the electromagnetic energy of molecular vibration. The energy band is defined for convenience as the near infrared (0.78-2.50 microns), the infrared (or mid-infrared) 2.50-40,0 microns, and the far infrared (40.0-1000 microns). However, even though official standards, textbooks, and the scientific literature generally state that the NIR spectral region extends from 780-2500 nanometers (12821-4000 cm-1), a simple set of liquid phase hydrocarbon spectra demonstrates that the vibrational information characterized by the harmonic vibrations of the C-H stretch fundamental and their corresponding combination bands occurs from approximately 690-3000 nm. The predominant near-infrared spectral features include: the methyl C-H stretching vibrations, methylene C-H stretching vibrations, aromatic C-H stretching vibrations, and O-H stretching vibrations. Minor but still important spectral features include: methoxy C-H stretching, carbonyl associated C-H stretching; N-H from primary amides, secondary amides (both alkyl, and aryl group associations), N-H from primary, secondary, and tertiary amines, and N-H from amine salts.

Qualitative and quantitative near infrared (NIR) spectroscopic methods typically require the application of multivariate calibration algorithms and statistical methods (i.e. chemometrics) to model NIR spectral response to chemical or physical properties of the samples used for calibration. The NIR method relies on the spectra-structure correlations existing between a measured spectral response caused by the harmonics of the fundamental vibrations occurring at infrared frequencies. These harmonic vibrations occur at unique frequencies depending upon the quantity of absorber (analyte), type of absorbing molecules present within the sample, and the sample thickness. Quantitative methods are possible where changes in the response of the near infrared spectrometer are proportional to changes in the concentration of chemical components, or in the physical characteristics (scattering/absorptive properties) of samples undergoing analysis

Near infrared spectroscopy is used where multicomponent molecular vibrational analysis is required in the presence of interfering substances. The near infrared spectra consist of overtones and combination bands of the fundamental molecular absorptions found in the mid infrared region. Near infrared spectra consist of generally overlapping vibrational bands that may appear non-specific and poorly resolved. The use of chemometric mathematical data processing and multiple harmonics can be used to calibrate for qualitative of quantitative analysis despite these apparent spectroscopic limitations. Traditional near infrared spectroscopy has been most often used for analysis of lignin polymers (2270 nm), paraffins and long alkane chain polymers (2310 nm), glucose based polymers such as cellulose (2336 nm), amino acid polymers as proteins (2180 nm), carbohydrates (2100 nm), and moisture (1440 and 1940 nm). When analyzing synthetic and natural materials NIR spectroscopy has shown unprecedented industrial success in multiple applications. The basic uses of near infrared spectroscopy have been for process control, quality assessment, identification of raw materials and process byproducts, and chemical quantitative analysis of complex mixtures.

Note that a near infrared spectrum consists in the convolution of the measuring instrument function with the unique optical characteristics of the sample being measured (i.e. the sample is an active optical element of the spectrometer). The reference values are those chemical or physical parameters to be predicted using the NIR spectroscopic measurements. A spectrum may, or may not, contain information related to the sample chemistry measured using any specific reference method. Spectra-structure correlation provides a basis for the establishment of a known cause and effect relationship between instrument response and reference (analyte) data, in order to provide a more scientific basis for multivariate-based near infrared spectroscopy. When performing multivariate calibrations, analytically valid calibration models require a relationship between X (the instrument response data or spectrum), and Y (the reference data). The use of probability alone tells us only if X and Y 'appear' to be related. If no cause-effect relationship exists between spectra-structure correlation and reference values the model will have no true predictive importance. Thus, knowledge of cause and effect creates a basis for scientific decision-making.

Factors affecting the integrity of the teaching samples used to calibrate spectrophotometers for individual NIR applications include the variations in sample chemistry, the physical condition of samples, and the measurement conditions. Teaching Sets must represent several sample 'spaces' to include: compositional space, instrument space, and measurement condition (sample handling and presentation) space. Interpretive spectroscopy is a key intellectual process in approaching NIR measurements if one is to achieve an analytical understanding of these measurements.

Near-infrared (NIR) spectroscopy has been employed for the characterization of products derived from petroleum, such as gasoline and diesel fuel, with considerable success. The intrinsic capacity of the NIR spectrum to obtain information on the different types of C-H bonds as well as other chemical bonds of interest (such as S-H and N-H) has been proved to be valuable in the prediction of quality parameters such as octane number, ethanol content, MTBE (methyl tert-butyl ether) content, distillation points, Reid vapor pressure and aromatic and saturated contents in gasoline. The information present in the NIR spectrum can be successfully applied to assign quality parameters for gas oil fractions such as cetane number, pour point, cloud point and aniline point.

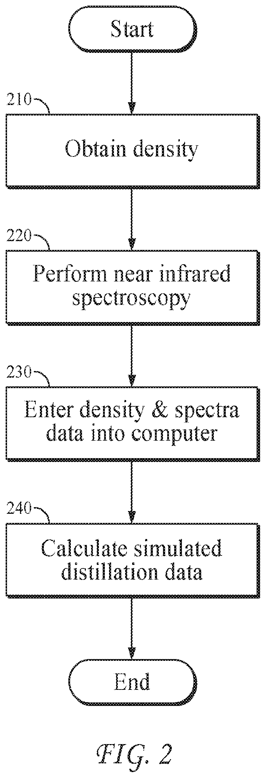

FIG. 2 shows a process flowchart of steps in a method according to one embodiment herein. In step 210, a crude oil sample is weighed and its density obtained. In step 220, crude oils were analyzed by near infrared spectroscopy, e.g., in accordance with the instructions of the equipment manufacturer. No dilution or special preparation is required.

In step 230, the density and spectra data are entered into a computer.

In step 240, the distillation temperature at a given distillation weight percentage is calculated as a function of the wavenumber at known weight percent absorbance values and the density.

Equation (1) is used to calculate and assign a distillation temperature for a given distillation weight percentage:

$$T_{DT} = K_{SD} + X1_{SD}\left(\frac{1}{NIRWN}\right) + X2_{SD}\left(\frac{1}{DEN}\right) + X3_{SD}\left(\frac{1}{NIRWN^2}\right) + X4_{SD}\left(\frac{1}{DEN^2}\right) + X5_{SD}\left(\frac{1}{NIRWN*DEN}\right)$$

$$X6_{SD}\left(\frac{1}{NIRWN^3}\right) + X7_{SD}\left(\frac{1}{DEN^3}\right) + X8_{SD}\left(\frac{1}{NIRWN^2*DEN}\right) + X9_{SD}\left(\frac{1}{DEN^2*NIRWN}\right)$$

$$(1);$$

where:

DT is the distillation weight percentage, $K_{SD}$, $X1_{SD}$, $X2_{SD}$, $X3_{SD}$, $X4_{SD}$, $X5_{SD}$, $X6_{SD}$, $X7_{SD}$, $X8_{SD}$ and $X9_{SD}$ are constants, DEN is the density of the sample (kg/L), and NIRWN is the wavenumber at DT.

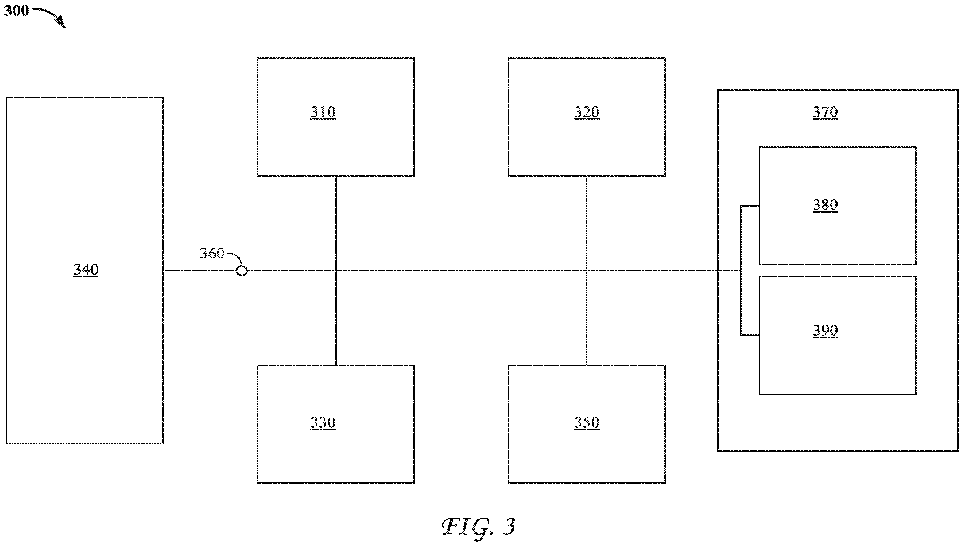

An exemplary block diagram of a computer system 300 by which simulated distillation data calculation modules can be implemented is shown in FIG. 3. Computer system 300 includes a processor 310, such as a central processing unit, an input/output interface 320 and support circuitry 330. In certain embodiments, where the computer 300 requires direct human interaction, a display 340 and an input device 350 such as a keyboard, mouse or pointer are also provided. The display 340, input device 350, processor 310, input/output interface 320 and support circuitry 330 are shown connected to a bus 360 which also connects to a memory unit 370. Memory 370 includes program storage memory 380 and data storage memory 390. Note that while computer 300 is depicted with the direct human interface components of display 340 and input device 350, programming of modules and importation and exportation of data can also be accomplished over the interface 320, for instance, where the computer 300 is connected to a network and the programming and display operations occur on another associated computer, or via a detachable input device, as are well known in the art for interfacing programmable logic controllers.

Program storage memory 380 and data storage memory 390 can each comprise volatile (RAM) and non-volatile (ROM) memory units and can also comprise hard disk and backup storage capacity, and both program storage memory 380 and data storage memory 390 can be embodied in a single memory device or separated in plural memory devices. Program storage memory 380 stores software program modules and associated data, and in particular stores calculation module(s) for obtaining the simulated distillation data. Data storage memory 390 stores data used and/or generated by the one or more modules of the present invention, including density of the oil sample, NIR spectroscopy data or portions thereof used by the one or more modules of the present system, and calculated data generated by the one or more modules of the present system.

The calculated and assigned results in accordance with the systems and methods herein are displayed, audibly outputted, printed, and/or stored to memory for use as described herein.

It is to be appreciated that the computer system 300 can be any general or special purpose computer such as a personal computer, minicomputer, workstation, mainframe, a dedicated controller such as a programmable logic controller, or a combination thereof. While the computer system 300 is shown, for illustration purposes, as a single computer unit, the system can comprise a group/farm of computers which can be scaled depending on the processing load and database size, e.g., the total number of samples that are processed and results maintained on the system.

The computer system 300 can serve as a common multi-tasking computer.

The computing device 300 preferably supports an operating system, for example, stored in program storage memory 390 and executed by the processor 310 from volatile memory. According to the present system and method, the operating system contains instructions for interfacing the device 300 to the calculation module(s). According to an embodiment of the invention, the operating system contains instructions for interfacing computer system 300 to the Internet and/or to private networks.

## Example

A sample of Arabian medium crude with a density of 0.8828 Kg/l was analyzed by near infrared spectroscopy. The spectra data, which was obtained in the wavenumber range 4,000-12,821, is presented in Table 3 and is shown in FIG. 1 as the sample with an API gravity of 28.8°. Cumulative near infrared absorbance of the sample was calculated from the near infrared spectroscopy data and normalized; a summary of absorbances versus wavenumber was obtained using interpolation and/or numerical methods as follows:

5 W%= 1.2091*1e-4 cm-1; 10 W%= 1.1721*1e-4 cm-1; 20 W%= 1.0978*1e-4 cm-1;30 W%= 1.0185*1e-4 cm-1; 40 W%= 0.9405*1e-4 cm-1; 50 W%= 0.8747*1e-4 cm-1; 60 W%= 0.8161*1e-4 cm-1; 70 W%= 0.7407*1e-4 cm-1; 80 W%= 0.6268*1e-4 cm-1.

The simulated distillation curve was obtained using Equation 1. The following constant values were used to predict the temperature at 50 W% point, obtained by linear regression:

$K_{SD}$ = 3.74721865E+04
$X1_{SD}$ = 5.72117464E+04
$X2_{SD}$ = 4.50736562E+04
$X3_{SD}$ = 5.83590589E+03
$X4_{SD}$ = 1.79172881E+04
$X5_{SD}$ = 1.18035802E+05
$X6_{SD}$ = 3.99969439E+02
$X7_{SD}$ = 2.37278141E+04
$X8_{SD}$ = 3.18900766E+03
$X9_{SD}$ = 5.63368697E+04

Using the above constants at a distillation weight percentage DT of 50%, the simulated distillation temperature at 50% distillation weight percentage is calculated and assigned as 412.4 °C. The temperatures at 0.5, 5, 10, 20, 30, 40, 50, 60, 70, 80 W% points are calculated and compared with the actual data, and a perfect fit was obtained.

In alternate embodiments, the present invention can be implemented as a computer program product for use with a computerized computing system. Those skilled in the art will readily appreciate that programs defining the functions of the present invention can be written in any appropriate programming language and delivered to a computer in any form, including but not limited to: (a) information permanently stored on non-writeable storage media (e.g., read-only memory devices such as ROMs or CD-ROM disks); (b) information alterably stored on writeable storage media (e.g., floppy disks and hard drives); and/or (c) information conveyed to a computer through communication media, such as a local area network, a telephone network, or a public network such as the Internet. When carrying computer readable instructions that implement the present invention methods, such computer readable media represent alternate embodiments of the present invention.

As generally illustrated herein, the system embodiments can incorporate a variety of computer readable media that comprise a computer usable medium having computer readable code means embodied therein. One skilled in the art will recognize that the software associated with the various processes described can be embodied in a wide variety of computer accessible media from which the software is loaded and activated. Pursuant to *In re Beauregard,* 35 USPQ2d 1383 (U.S. Patent 5,710,578), the present invention contemplates and includes this type of computer readable media within the scope of the invention. In certain embodiments, pursuant to *In re Nuijten,* 500 F.3d 1346 (Fed. Cir. 2007) (U.S. Patent Application Serial Number 09/211,928), the scope of the present claims is limited to computer readable media, wherein the media is both tangible and non-transitory.

The system and method of the present invention have been described above and with reference to the attached figure; however, modifications will be apparent to those of ordinary skill in the art and the scope of protection for the invention is to be defined by the claims that follow.

Table 3

| cm⁻¹ | Absorb. | cm⁻¹ | Absorb. | cm⁻¹ | Absorb. | cm⁻¹ | Absorb. | cm⁻¹ | Absorb. |
|---|---|---|---|---|---|---|---|---|---|
| 12493 | 3.61 | 10792 | 3.64 | 9091 | 4.37 | 7390 | 3.21 | 5689 | 3.18 |
| 12489 | 3.56 | 10788 | 3.65 | 9087 | 4.28 | 7386 | 3.21 | 5685 | 3.2 |
| 12485 | 3.52 | 10784 | 3.65 | 9084 | 4.25 | 7383 | 3.21 | 5682 | 3.23 |
| 12482 | 3.48 | 10781 | 3.62 | 9080 | 4.3 | 7379 | 3.21 | 5678 | 3.24 |
| 12478 | 3.51 | 10777 | 3.61 | 9076 | 4.42 | 7375 | 3.2 | 5674 | 3.24 |
| 12474 | 3.56 | 10773 | 3.62 | 9072 | 4.48 | 7371 | 3.2 | 5670 | 3.23 |
| 12470 | 3.57 | 10769 | 3.64 | 9068 | 4.41 | 7367 | 3.2 | 5666 | 3.19 |
| 12466 | 3.56 | 10765 | 3.64 | 9064 | 4.36 | 7363 | 3.2 | 5662 | 3.15 |
| 12462 | 3.58 | 10761 | 3.67 | 9060 | 4.36 | 7359 | 3.21 | 5658 | 3.09 |
| 12458 | 3.6 | 10757 | 3.7 | 9057 | 4.37 | 7356 | 3.21 | 5655 | 3.03 |
| 12455 | 3.6 | 10754 | 3.68 | 9053 | 4.35 | 7352 | 3.21 | 5651 | 2.96 |
| 12451 | 3.65 | 10750 | 3.61 | 9049 | 4.36 | 7348 | 3.21 | 5647 | 2.9 |
| 12447 | 3.74 | 10746 | 3.58 | 9045 | 4.38 | 7344 | 3.21 | 5643 | 2.84 |
| 12443 | 3.72 | 10742 | 3.6 | 9041 | 4.45 | 7340 | 3.2 | 5639 | 2.78 |
| 12439 | 3.68 | 10738 | 3.62 | 9037 | 4.52 | 7336 | 3.2 | 5635 | 2.72 |
| 12435 | 3.67 | 10734 | 3.64 | 9033 | 4.51 | 7332 | 3.19 | 5631 | 2.66 |
| 12431 | 3.63 | 10730 | 3.66 | 9030 | 4.42 | 7329 | 3.19 | 5628 | 2.6 |
| 12428 | 3.6 | 10727 | 3.68 | 9026 | 4.34 | 7325 | 3.18 | 5624 | 2.54 |
| 12424 | 3.57 | 10723 | 3.68 | 9022 | 4.32 | 7321 | 3.18 | 5620 | 2.49 |
| 12420 | 3.51 | 10719 | 3.67 | 9018 | 4.34 | 7317 | 3.18 | 5616 | 2.44 |
| 12416 | 3.5 | 10715 | 3.66 | 9014 | 4.37 | 7313 | 3.18 | 5612 | 2.39 |
| 12412 | 3.57 | 10711 | 3.67 | 9010 | 4.41 | 7309 | 3.18 | 5608 | 2.35 |
| 12408 | 3.59 | 10707 | 3.66 | 9006 | 4.45 | 7305 | 3.17 | 5604 | 2.31 |
| 12404 | 3.54 | 10703 | 3.65 | 9003 | 4.44 | 7302 | 3.17 | 5601 | 2.28 |
| 12401 | 3.57 | 10700 | 3.64 | 8999 | 4.43 | 7298 | 3.17 | 5597 | 2.26 |
| 12397 | 3.68 | 10696 | 3.62 | 8995 | 4.49 | 7294 | 3.17 | 5593 | 2.24 |
| 12393 | 3.7 | 10692 | 3.6 | 8991 | 4.55 | 7290 | 3.17 | 5589 | 2.22 |
| 12389 | 3.63 | 10688 | 3.6 | 8987 | 4.57 | 7286 | 3.18 | 5585 | 2.21 |
| 12385 | 3.58 | 10684 | 3.61 | 8983 | 4.57 | 7282 | 3.18 | 5581 | 2.2 |
| 12381 | 3.58 | 10680 | 3.64 | 8979 | 4.58 | 7278 | 3.18 | 5577 | 2.2 |
| 12377 | 3.57 | 10676 | 3.67 | 8976 | 4.58 | 7275 | 3.18 | 5574 | 2.19 |
| 12374 | 3.57 | 10673 | 3.68 | 8972 | 4.55 | 7271 | 3.18 | 5570 | 2.18 |
| 12370 | 3.57 | 10669 | 3.68 | 8968 | 4.48 | 7267 | 3.18 | 5566 | 2.18 |
| 12366 | 3.56 | 10665 | 3.67 | 8964 | 4.46 | 7263 | 3.18 | 5562 | 2.17 |
| 12362 | 3.55 | 10661 | 3.65 | 8960 | 4.49 | 7259 | 3.19 | 5558 | 2.17 |
| 12358 | 3.56 | 10657 | 3.64 | 8956 | 4.52 | 7255 | 3.19 | 5554 | 2.16 |
| 12354 | 3.56 | 10653 | 3.65 | 8952 | 4.53 | 7251 | 3.19 | 5550 | 2.15 |
| 12350 | 3.54 | 10649 | 3.67 | 8949 | 4.55 | 7248 | 3.19 | 5547 | 2.15 |

(continued)

| cm⁻¹ | Absorb. | cm⁻¹ | Absorb. | cm⁻¹ | Absorb. | cm⁻¹ | Absorb. | cm⁻¹ | Absorb. |
|---|---|---|---|---|---|---|---|---|---|
| 12347 | 3.51 | 10646 | 3.69 | 8945 | 4.59 | 7244 | 3.19 | 5543 | 2.14 |
| 12343 | 3.5 | 10642 | 3.69 | 8941 | 4.62 | 7240 | 3.19 | 5539 | 2.13 |
| 12339 | 3.53 | 10638 | 3.69 | 8937 | 4.65 | 7236 | 3.19 | 5535 | 2.13 |
| 12335 | 3.6 | 10634 | 3.68 | 8933 | 4.69 | 7232 | 3.19 | 5531 | 2.12 |
| 12331 | 3.64 | 10630 | 3.66 | 8929 | 4.73 | 7228 | 3.19 | 5527 | 2.12 |
| 12327 | 3.59 | 10626 | 3.64 | 8925 | 4.77 | 7224 | 3.19 | 5523 | 2.12 |
| 12323 | 3.54 | 10622 | 3.66 | 8922 | 4.86 | 7221 | 3.19 | 5520 | 2.12 |
| 12320 | 3.57 | 10619 | 3.68 | 8918 | 4.84 | 7217 | 3.19 | 5516 | 2.12 |
| 12316 | 3.68 | 10615 | 3.66 | 8914 | 4.75 | 7213 | 3.19 | 5512 | 2.12 |
| 12312 | 3.68 | 10611 | 3.62 | 8910 | 4.82 | 7209 | 3.2 | 5508 | 2.12 |
| 12308 | 3.57 | 10607 | 3.61 | 8906 | 4.98 | 7205 | 3.2 | 5504 | 2.12 |
| 12304 | 3.5 | 10603 | 3.64 | 8902 | 5.07 | 7201 | 3.2 | 5500 | 2.12 |
| 12300 | 3.48 | 10599 | 3.7 | 8898 | 4.85 | 7197 | 3.2 | 5496 | 2.12 |
| 12296 | 3.5 | 10595 | 3.75 | 8895 | 4.72 | 7194 | 3.2 | 5493 | 2.11 |
| 12293 | 3.6 | 10592 | 3.75 | 8891 | 4.68 | 7190 | 3.21 | 5489 | 2.11 |
| 12289 | 3.66 | 10588 | 3.72 | 8887 | 4.79 | 7186 | 3.21 | 5485 | 2.1 |
| 12285 | 3.64 | 10584 | 3.71 | 8883 | 5.05 | 7182 | 3.2 | 5481 | 2.1 |
| 12281 | 3.69 | 10580 | 3.71 | 8879 | 4.87 | 7178 | 3.19 | 5477 | 2.09 |
| 12277 | 3.7 | 10576 | 3.71 | 8875 | 4.98 | 7174 | 3.18 | 5473 | 2.08 |
| 12273 | 3.62 | 10572 | 3.71 | 8871 | 5.11 | 7170 | 3.17 | 5469 | 2.08 |
| 12269 | 3.56 | 10568 | 3.68 | 8868 | 5.22 | 7167 | 3.16 | 5466 | 2.07 |
| 12266 | 3.53 | 10565 | 3.64 | 8864 | 5.1 | 7163 | 3.14 | 5462 | 2.06 |
| 12262 | 3.56 | 10561 | 3.63 | 8860 | 4.98 | 7159 | 3.12 | 5458 | 2.04 |
| 12258 | 3.63 | 10557 | 3.64 | 8856 | 4.93 | 7155 | 3.11 | 5454 | 2.03 |
| 12254 | 3.74 | 10553 | 3.65 | 8852 | 4.99 | 7151 | 3.1 | 5450 | 2.02 |
| 12250 | 3.88 | 10549 | 3.66 | 8848 | 5.02 | 7147 | 3.08 | 5446 | 2.01 |
| 12246 | 3.79 | 10545 | 3.67 | 8844 | 4.72 | 7143 | 3.07 | 5442 | 1.99 |
| 12242 | 3.7 | 10541 | 3.69 | 8841 | 4.65 | 7140 | 3.06 | 5439 | 1.98 |
| 12239 | 3.64 | 10538 | 3.69 | 8837 | 4.74 | 7136 | 3.05 | 5435 | 1.97 |
| 12235 | 3.55 | 10534 | 3.69 | 8833 | 4.88 | 7132 | 3.04 | 5431 | 1.95 |
| 12231 | 3.48 | 10530 | 3.7 | 8829 | 4.92 | 7128 | 3.04 | 5427 | 1.94 |
| 12227 | 3.47 | 10526 | 3.71 | 8825 | 4.94 | 7124 | 3.03 | 5423 | 1.92 |
| 12223 | 3.46 | 10522 | 3.71 | 8821 | 4.85 | 7120 | 3.03 | 5419 | 1.91 |
| 12219 | 3.45 | 10518 | 3.7 | 8817 | 4.73 | 7116 | 3.02 | 5415 | 1.89 |
| 12215 | 3.44 | 10514 | 3.68 | 8814 | 4.81 | 7113 | 3.02 | 5412 | 1.88 |
| 12212 | 3.42 | 10511 | 3.67 | 8810 | 5.14 | 7109 | 3.02 | 5408 | 1.86 |
| 12208 | 3.47 | 10507 | 3.68 | 8806 | 5.04 | 7105 | 3.02 | 5404 | 1.84 |
| 12204 | 3.61 | 10503 | 3.71 | 8802 | 4.98 | 7101 | 3.02 | 5400 | 1.83 |

(continued)

| cm⁻¹ | Absorb. | cm⁻¹ | Absorb. | cm⁻¹ | Absorb. | cm⁻¹ | Absorb. | cm⁻¹ | Absorb. |
|---|---|---|---|---|---|---|---|---|---|
| 12200 | 3.73 | 10499 | 3.75 | 8798 | 4.94 | 7097 | 3.02 | 5396 | 1.81 |
| 12196 | 3.67 | 10495 | 3.75 | 8794 | 5.18 | 7093 | 3.02 | 5392 | 1.8 |
| 12192 | 3.6 | 10491 | 3.72 | 8790 | 5.24 | 7089 | 3.02 | 5388 | 1.78 |
| 12188 | 3.6 | 10487 | 3.7 | 8787 | 5.23 | 7086 | 3.02 | 5385 | 1.77 |
| 12185 | 3.61 | 10484 | 3.72 | 8783 | 5.04 | 7082 | 3.02 | 5381 | 1.76 |
| 12181 | 3.59 | 10480 | 3.75 | 8779 | 4.91 | 7078 | 3.02 | 5377 | 1.74 |
| 12177 | 3.61 | 10476 | 3.74 | 8775 | 4.95 | 7074 | 3.01 | 5373 | 1.73 |
| 12173 | 3.66 | 10472 | 3.71 | 8771 | 5.01 | 7070 | 3 | 5369 | 1.72 |
| 12169 | 3.65 | 10468 | 3.72 | 8767 | 4.97 | 7066 | 2.99 | 5365 | 1.72 |
| 12165 | 3.62 | 10464 | 3.73 | 8763 | 4.89 | 7062 | 2.97 | 5361 | 1.71 |
| 12161 | 3.62 | 10460 | 3.72 | 8760 | 4.83 | 7059 | 2.96 | 5358 | 1.7 |
| 12158 | 3.59 | 10457 | 3.72 | 8756 | 4.84 | 7055 | 2.95 | 5354 | 1.7 |
| 12154 | 3.55 | 10453 | 3.71 | 8752 | 4.89 | 7051 | 2.93 | 5350 | 1.69 |
| 12150 | 3.56 | 10449 | 3.7 | 8748 | 4.8 | 7047 | 2.92 | 5346 | 1.69 |
| 12146 | 3.6 | 10445 | 3.72 | 8744 | 4.82 | 7043 | 2.91 | 5342 | 1.68 |
| 12142 | 3.59 | 10441 | 3.75 | 8740 | 5.06 | 7039 | 2.9 | 5338 | 1.68 |
| 12138 | 3.57 | 10437 | 3.76 | 8736 | 5.1 | 7035 | 2.89 | 5334 | 1.68 |
| 12134 | 3.62 | 10433 | 3.73 | 8733 | 4.99 | 7032 | 2.88 | 5331 | 1.68 |
| 12131 | 3.7 | 10430 | 3.72 | 8729 | 4.98 | 7028 | 2.88 | 5327 | 1.67 |
| 12127 | 3.73 | 10426 | 3.7 | 8725 | 4.95 | 7024 | 2.87 | 5323 | 1.67 |
| 12123 | 3.7 | 10422 | 3.67 | 8721 | 4.87 | 7020 | 2.86 | 5319 | 1.67 |
| 12119 | 3.62 | 10418 | 3.68 | 8717 | 4.93 | 7016 | 2.86 | 5315 | 1.67 |
| 12115 | 3.57 | 10414 | 3.7 | 8713 | 4.93 | 7012 | 2.85 | 5311 | 1.66 |
| 12111 | 3.59 | 10410 | 3.74 | 8709 | 4.84 | 7008 | 2.84 | 5307 | 1.66 |
| 12107 | 3.64 | 10406 | 3.78 | 8706 | 5.04 | 7005 | 2.84 | 5304 | 1.66 |
| 12104 | 3.62 | 10403 | 3.77 | 8702 | 5.14 | 7001 | 2.83 | 5300 | 1.65 |
| 12100 | 3.57 | 10399 | 3.75 | 8698 | 5.07 | 6997 | 2.83 | 5296 | 1.65 |
| 12096 | 3.55 | 10395 | 3.73 | 8694 | 5.2 | 6993 | 2.83 | 5292 | 1.65 |
| 12092 | 3.54 | 10391 | 3.74 | 8690 | 5.17 | 6989 | 2.82 | 5288 | 1.65 |
| 12088 | 3.53 | 10387 | 3.74 | 8686 | 5.13 | 6985 | 2.82 | 5284 | 1.64 |
| 12084 | 3.52 | 10383 | 3.74 | 8682 | 5.54 | 6981 | 2.81 | 5280 | 1.64 |
| 12080 | 3.53 | 10379 | 3.72 | 8679 | 5.32 | 6978 | 2.81 | 5277 | 1.64 |
| 12077 | 3.6 | 10376 | 3.72 | 8675 | 5.11 | 6974 | 2.8 | 5273 | 1.64 |
| 12073 | 3.72 | 10372 | 3.75 | 8671 | 5.29 | 6970 | 2.79 | 5269 | 1.63 |
| 12069 | 3.76 | 10368 | 3.76 | 8667 | 5.02 | 6966 | 2.78 | 5265 | 1.63 |
| 12065 | 3.74 | 10364 | 3.77 | 8663 | 4.72 | 6962 | 2.78 | 5261 | 1.62 |
| 12061 | 3.72 | 10360 | 3.77 | 8659 | 4.72 | 6958 | 2.77 | 5257 | 1.62 |
| 12057 | 3.71 | 10356 | 3.76 | 8655 | 4.85 | 6954 | 2.76 | 5253 | 1.62 |

(continued)

| cm⁻¹ | Absorb. | cm⁻¹ | Absorb. | cm⁻¹ | Absorb. | cm⁻¹ | Absorb. | cm⁻¹ | Absorb. |
|---|---|---|---|---|---|---|---|---|---|
| 12053 | 3.72 | 10352 | 3.75 | 8652 | 4.95 | 6951 | 2.75 | 5250 | 1.61 |
| 12050 | 3.77 | 10349 | 3.75 | 8648 | 4.98 | 6947 | 2.74 | 5246 | 1.61 |
| 12046 | 3.78 | 10345 | 3.77 | 8644 | 4.99 | 6943 | 2.74 | 5242 | 1.61 |
| 12042 | 3.7 | 10341 | 3.77 | 8640 | 4.99 | 6939 | 2.73 | 5238 | 1.6 |
| 12038 | 3.62 | 10337 | 3.75 | 8636 | 4.97 | 6935 | 2.72 | 5234 | 1.6 |
| 12034 | 3.64 | 10333 | 3.72 | 8632 | 4.96 | 6931 | 2.71 | 5230 | 1.6 |
| 12030 | 3.8 | 10329 | 3.68 | 8628 | 4.93 | 6927 | 2.7 | 5226 | 1.6 |
| 12026 | 3.98 | 10325 | 3.68 | 8625 | 4.88 | 6924 | 2.7 | 5223 | 1.59 |
| 12023 | 3.98 | 10322 | 3.72 | 8621 | 4.98 | 6920 | 2.69 | 5219 | 1.59 |
| 12019 | 3.87 | 10318 | 3.77 | 8617 | 5.51 | 6916 | 2.68 | 5215 | 1.59 |
| 12015 | 3.77 | 10314 | 3.79 | 8613 | 5.23 | 6912 | 2.67 | 5211 | 1.59 |
| 12011 | 3.7 | 10310 | 3.75 | 8609 | 5.12 | 6908 | 2.67 | 5207 | 1.58 |
| 12007 | 3.71 | 10306 | 3.73 | 8605 | 5.18 | 6904 | 2.66 | 5203 | 1.58 |
| 12003 | 3.79 | 10302 | 3.76 | 8601 | 5.05 | 6900 | 2.65 | 5199 | 1.58 |
| 11999 | 3.89 | 10298 | 3.79 | 8598 | 4.87 | 6897 | 2.64 | 5196 | 1.57 |
| 11996 | 3.88 | 10295 | 3.76 | 8594 | 4.85 | 6893 | 2.63 | 5192 | 1.57 |
| 11992 | 3.76 | 10291 | 3.74 | 8590 | 4.85 | 6889 | 2.63 | 5188 | 1.57 |
| 11988 | 3.66 | 10287 | 3.76 | 8586 | 5.01 | 6885 | 2.62 | 5184 | 1.56 |
| 11984 | 3.63 | 10283 | 3.79 | 8582 | 5.73 | 6881 | 2.61 | 5180 | 1.56 |
| 11980 | 3.65 | 10279 | 3.79 | 8578 | 4.99 | 6877 | 2.6 | 5176 | 1.56 |
| 11976 | 3.7 | 10275 | 3.78 | 8574 | 4.93 | 6873 | 2.59 | 5172 | 1.55 |
| 11972 | 3.78 | 10271 | 3.78 | 8571 | 4.96 | 6870 | 2.59 | 5169 | 1.55 |
| 11969 | 3.83 | 10268 | 3.78 | 8567 | 4.96 | 6866 | 2.58 | 5165 | 1.55 |
| 11965 | 3.78 | 10264 | 3.77 | 8563 | 5.04 | 6862 | 2.57 | 5161 | 1.54 |
| 11961 | 3.69 | 10260 | 3.76 | 8559 | 5.13 | 6858 | 2.57 | 5157 | 1.54 |
| 11957 | 3.64 | 10256 | 3.75 | 8555 | 5.1 | 6854 | 2.56 | 5153 | 1.54 |
| 11953 | 3.64 | 10252 | 3.74 | 8551 | 4.93 | 6850 | 2.56 | 5149 | 1.53 |
| 11949 | 3.69 | 10248 | 3.76 | 8547 | 4.8 | 6846 | 2.55 | 5145 | 1.53 |
| 11945 | 3.74 | 10244 | 3.79 | 8544 | 4.75 | 6843 | 2.54 | 5142 | 1.53 |
| 11942 | 3.73 | 10241 | 3.81 | 8540 | 4.76 | 6839 | 2.54 | 5138 | 1.53 |
| 11938 | 3.73 | 10237 | 3.77 | 8536 | 4.87 | 6835 | 2.53 | 5134 | 1.52 |
| 11934 | 3.8 | 10233 | 3.72 | 8532 | 5 | 6831 | 2.53 | 5130 | 1.52 |
| 11930 | 3.87 | 10229 | 3.7 | 8528 | 4.92 | 6827 | 2.52 | 5126 | 1.52 |
| 11926 | 3.89 | 10225 | 3.73 | 8524 | 4.93 | 6823 | 2.52 | 5122 | 1.52 |
| 11922 | 3.9 | 10221 | 3.77 | 8520 | 5.1 | 6819 | 2.51 | 5118 | 1.51 |
| 11918 | 3.95 | 10217 | 3.79 | 8517 | 5.32 | 6816 | 2.51 | 5115 | 1.51 |
| 11915 | 4.06 | 10214 | 3.76 | 8513 | 5.09 | 6812 | 2.5 | 5111 | 1.51 |
| 11911 | 4.15 | 10210 | 3.73 | 8509 | 4.82 | 6808 | 2.49 | 5107 | 1.51 |

(continued)

| cm⁻¹ | Absorb. | cm⁻¹ | Absorb. | cm⁻¹ | Absorb. | cm⁻¹ | Absorb. | cm⁻¹ | Absorb. |
|---|---|---|---|---|---|---|---|---|---|
| 11907 | 4.06 | 10206 | 3.73 | 8505 | 4.77 | 6804 | 2.49 | 5103 | 1.51 |
| 11903 | 3.94 | 10202 | 3.76 | 8501 | 4.84 | 6800 | 2.48 | 5099 | 1.51 |
| 11899 | 3.88 | 10198 | 3.77 | 8497 | 4.93 | 6796 | 2.48 | 5095 | 1.5 |
| 11895 | 3.9 | 10194 | 3.77 | 8493 | 4.81 | 6792 | 2.47 | 5091 | 1.5 |
| 11891 | 3.92 | 10190 | 3.78 | 8490 | 4.71 | 6789 | 2.46 | 5088 | 1.5 |
| 11888 | 3.92 | 10187 | 3.78 | 8486 | 4.74 | 6785 | 2.45 | 5084 | 1.5 |
| 11884 | 4.02 | 10183 | 3.78 | 8482 | 4.77 | 6781 | 2.45 | 5080 | 1.5 |
| 11880 | 4.12 | 10179 | 3.76 | 8478 | 4.77 | 6777 | 2.44 | 5076 | 1.49 |
| 11876 | 4.15 | 10175 | 3.75 | 8474 | 4.96 | 6773 | 2.44 | 5072 | 1.49 |
| 11872 | 4.16 | 10171 | 3.75 | 8470 | 5.11 | 6769 | 2.43 | 5068 | 1.49 |
| 11868 | 4.13 | 10167 | 3.78 | 8466 | 5.21 | 6765 | 2.42 | 5064 | 1.49 |
| 11864 | 4.03 | 10163 | 3.78 | 8463 | 5.07 | 6762 | 2.42 | 5061 | 1.48 |
| 11861 | 4.03 | 10160 | 3.75 | 8459 | 5.16 | 6758 | 2.41 | 5057 | 1.48 |
| 11857 | 4.28 | 10156 | 3.72 | 8455 | 5.11 | 6754 | 2.41 | 5053 | 1.48 |
| 11853 | 4.47 | 10152 | 3.73 | 8451 | 4.99 | 6750 | 2.4 | 5049 | 1.48 |
| 11849 | 4.29 | 10148 | 3.77 | 8447 | 4.92 | 6746 | 2.39 | 5045 | 1.48 |
| 11845 | 4.1 | 10144 | 3.8 | 8443 | 4.86 | 6742 | 2.39 | 5041 | 1.48 |
| 11841 | 4.16 | 10140 | 3.78 | 8439 | 4.93 | 6738 | 2.38 | 5037 | 1.48 |
| 11837 | 4.25 | 10136 | 3.76 | 8436 | 5.09 | 6735 | 2.38 | 5034 | 1.48 |
| 11834 | 4 | 10133 | 3.75 | 8432 | 5.09 | 6731 | 2.37 | 5030 | 1.47 |
| 11830 | 3.96 | 10129 | 3.74 | 8428 | 4.88 | 6727 | 2.37 | 5026 | 1.47 |
| 11826 | 4.01 | 10125 | 3.73 | 8424 | 4.85 | 6723 | 2.36 | 5022 | 1.47 |
| 11822 | 4.01 | 10121 | 3.71 | 8420 | 4.85 | 6719 | 2.36 | 5018 | 1.47 |
| 11818 | 4.01 | 10117 | 3.69 | 8416 | 4.85 | 6715 | 2.35 | 5014 | 1.47 |
| 11814 | 4.01 | 10113 | 3.69 | 8412 | 4.89 | 6711 | 2.34 | 5010 | 1.47 |
| 11810 | 4.05 | 10109 | 3.72 | 8409 | 5.09 | 6708 | 2.34 | 5007 | 1.47 |
| 11807 | 4.12 | 10106 | 3.73 | 8405 | 5.13 | 6704 | 2.33 | 5003 | 1.47 |
| 11803 | 4.17 | 10102 | 3.73 | 8401 | 5.06 | 6700 | 2.33 | 4999 | 1.47 |
| 11799 | 4.24 | 10098 | 3.74 | 8397 | 5.13 | 6696 | 2.32 | 4995 | 1.47 |
| 11795 | 4.37 | 10094 | 3.77 | 8393 | 5.04 | 6692 | 2.32 | 4991 | 1.47 |
| 11791 | 4.18 | 10090 | 3.77 | 8389 | 4.97 | 6688 | 2.31 | 4987 | 1.47 |
| 11787 | 4.09 | 10086 | 3.73 | 8385 | 5.11 | 6684 | 2.31 | 4983 | 1.47 |
| 11783 | 4.3 | 10082 | 3.71 | 8382 | 5.1 | 6681 | 2.3 | 4980 | 1.47 |
| 11780 | 4.27 | 10079 | 3.73 | 8378 | 5.06 | 6677 | 2.3 | 4976 | 1.47 |
| 11776 | 4.19 | 10075 | 3.75 | 8374 | 5.15 | 6673 | 2.29 | 4972 | 1.47 |
| 11772 | 4.12 | 10071 | 3.75 | 8370 | 5.08 | 6669 | 2.28 | 4968 | 1.46 |
| 11768 | 4.18 | 10067 | 3.73 | 8366 | 4.98 | 6665 | 2.28 | 4964 | 1.46 |
| 11764 | 4.13 | 10063 | 3.7 | 8362 | 4.88 | 6661 | 2.27 | 4960 | 1.45 |

(continued)

| cm⁻¹ | Absorb. | cm⁻¹ | Absorb. | cm⁻¹ | Absorb. | cm⁻¹ | Absorb. | cm⁻¹ | Absorb. |
|---|---|---|---|---|---|---|---|---|---|
| 11760 | 4.04 | 10059 | 3.71 | 8358 | 4.8 | 6657 | 2.27 | 4956 | 1.45 |
| 11756 | 4.05 | 10055 | 3.73 | 8355 | 4.81 | 6654 | 2.26 | 4953 | 1.44 |
| 11753 | 4.05 | 10052 | 3.76 | 8351 | 4.97 | 6650 | 2.26 | 4949 | 1.44 |
| 11749 | 4.15 | 10048 | 3.76 | 8347 | 5.11 | 6646 | 2.25 | 4945 | 1.43 |
| 11745 | 4.25 | 10044 | 3.76 | 8343 | 5.15 | 6642 | 2.25 | 4941 | 1.43 |
| 11741 | 4.26 | 10040 | 3.77 | 8339 | 5.36 | 6638 | 2.24 | 4937 | 1.42 |
| 11737 | 4.15 | 10036 | 3.78 | 8335 | 5.12 | 6634 | 2.23 | 4933 | 1.42 |
| 11733 | 4.17 | 10032 | 3.78 | 8331 | 5.03 | 6630 | 2.23 | 4929 | 1.41 |
| 11729 | 4.32 | 10028 | 3.78 | 8328 | 5.05 | 6627 | 2.22 | 4926 | 1.41 |
| 11726 | 4.35 | 10025 | 3.79 | 8324 | 5.1 | 6623 | 2.22 | 4922 | 1.4 |
| 11722 | 4.32 | 10021 | 3.81 | 8320 | 5.05 | 6619 | 2.21 | 4918 | 1.4 |
| 11718 | 4.21 | 10017 | 3.82 | 8316 | 5.14 | 6615 | 2.21 | 4914 | 1.39 |
| 11714 | 4.03 | 10013 | 3.8 | 8312 | 5.12 | 6611 | 2.2 | 4910 | 1.39 |
| 11710 | 4.08 | 10009 | 3.79 | 8308 | 5.05 | 6607 | 2.2 | 4906 | 1.39 |
| 11706 | 4.12 | 10005 | 3.82 | 8304 | 5.2 | 6603 | 2.19 | 4902 | 1.38 |
| 11702 | 4.02 | 10002 | 3.88 | 8301 | 4.99 | 6600 | 2.19 | 4899 | 1.38 |
| 11699 | 3.96 | 9998 | 3.89 | 8297 | 4.94 | 6596 | 2.18 | 4895 | 1.38 |
| 11695 | 4.12 | 9994 | 3.86 | 8293 | 5.27 | 6592 | 2.18 | 4891 | 1.38 |
| 11691 | 4.32 | 9990 | 3.83 | 8289 | 5.18 | 6588 | 2.17 | 4887 | 1.37 |
| 11687 | 4.4 | 9986 | 3.8 | 8285 | 5.06 | 6584 | 2.17 | 4883 | 1.37 |
| 11683 | 4.23 | 9982 | 3.77 | 8281 | 5.11 | 6580 | 2.16 | 4879 | 1.37 |
| 11679 | 4.22 | 9978 | 3.75 | 8277 | 5.06 | 6576 | 2.16 | 4875 | 1.37 |
| 11675 | 4.29 | 9975 | 3.74 | 8274 | 4.98 | 6573 | 2.15 | 4872 | 1.36 |
| 11672 | 4.46 | 9971 | 3.75 | 8270 | 4.96 | 6569 | 2.15 | 4868 | 1.36 |
| 11668 | 4.58 | 9967 | 3.77 | 8266 | 4.97 | 6565 | 2.15 | 4864 | 1.35 |
| 11664 | 4.41 | 9963 | 3.77 | 8262 | 4.99 | 6561 | 2.14 | 4860 | 1.35 |
| 11660 | 4.19 | 9959 | 3.76 | 8258 | 5.01 | 6557 | 2.14 | 4856 | 1.35 |
| 11656 | 4.11 | 9955 | 3.75 | 8254 | 5.07 | 6553 | 2.13 | 4852 | 1.34 |
| 11652 | 4.1 | 9951 | 3.75 | 8250 | 5.24 | 6549 | 2.13 | 4848 | 1.34 |
| 11648 | 4.15 | 9948 | 3.76 | 8247 | 5.24 | 6546 | 2.12 | 4845 | 1.33 |
| 11645 | 4.27 | 9944 | 3.79 | 8243 | 5.14 | 6542 | 2.12 | 4841 | 1.33 |
| 11641 | 4.2 | 9940 | 3.8 | 8239 | 5.21 | 6538 | 2.11 | 4837 | 1.33 |
| 11637 | 4.02 | 9936 | 3.8 | 8235 | 4.98 | 6534 | 2.11 | 4833 | 1.32 |
| 11633 | 4.08 | 9932 | 3.78 | 8231 | 4.82 | 6530 | 2.11 | 4829 | 1.32 |
| 11629 | 4.14 | 9928 | 3.77 | 8227 | 4.8 | 6526 | 2.1 | 4825 | 1.32 |
| 11625 | 4.07 | 9924 | 3.78 | 8223 | 4.8 | 6522 | 2.1 | 4821 | 1.32 |
| 11621 | 4.05 | 9921 | 3.78 | 8220 | 4.73 | 6519 | 2.09 | 4818 | 1.32 |
| 11618 | 4.11 | 9917 | 3.77 | 8216 | 4.69 | 6515 | 2.09 | 4814 | 1.31 |

(continued)

| cm⁻¹ | Absorb. | cm⁻¹ | Absorb. | cm⁻¹ | Absorb. | cm⁻¹ | Absorb. | cm⁻¹ | Absorb. |
|---|---|---|---|---|---|---|---|---|---|
| 11614 | 4.11 | 9913 | 3.78 | 8212 | 4.8 | 6511 | 2.08 | 4810 | 1.31 |
| 11610 | 4.11 | 9909 | 3.78 | 8208 | 4.87 | 6507 | 2.08 | 4806 | 1.31 |
| 11606 | 4.07 | 9905 | 3.77 | 8204 | 4.87 | 6503 | 2.07 | 4802 | 1.31 |
| 11602 | 4.11 | 9901 | 3.77 | 8200 | 4.84 | 6499 | 2.07 | 4798 | 1.31 |
| 11598 | 4.21 | 9897 | 3.78 | 8196 | 4.78 | 6495 | 2.07 | 4794 | 1.31 |
| 11594 | 4.15 | 9894 | 3.78 | 8193 | 4.75 | 6492 | 2.06 | 4791 | 1.31 |
| 11591 | 4.05 | 9890 | 3.76 | 8189 | 4.77 | 6488 | 2.06 | 4787 | 1.31 |
| 11587 | 3.99 | 9886 | 3.74 | 8185 | 4.84 | 6484 | 2.05 | 4783 | 1.31 |
| 11583 | 3.98 | 9882 | 3.73 | 8181 | 4.83 | 6480 | 2.05 | 4779 | 1.31 |
| 11579 | 4.05 | 9878 | 3.74 | 8177 | 4.67 | 6476 | 2.04 | 4775 | 1.31 |
| 11575 | 4.15 | 9874 | 3.77 | 8173 | 4.57 | 6472 | 2.04 | 4771 | 1.31 |
| 11571 | 4.21 | 9870 | 3.78 | 8169 | 4.56 | 6468 | 2.03 | 4767 | 1.31 |
| 11567 | 4.21 | 9867 | 3.76 | 8166 | 4.65 | 6465 | 2.03 | 4764 | 1.31 |
| 11564 | 4.4 | 9863 | 3.76 | 8162 | 4.74 | 6461 | 2.02 | 4760 | 1.31 |
| 11560 | 4.54 | 9859 | 3.77 | 8158 | 4.73 | 6457 | 2.02 | 4756 | 1.31 |
| 11556 | 4.37 | 9855 | 3.77 | 8154 | 4.73 | 6453 | 2.01 | 4752 | 1.31 |
| 11552 | 4.18 | 9851 | 3.74 | 8150 | 4.77 | 6449 | 2.01 | 4748 | 1.3 |
| 11548 | 4.18 | 9847 | 3.73 | 8146 | 4.82 | 6445 | 2 | 4744 | 1.3 |
| 11544 | 4.2 | 9843 | 3.74 | 8142 | 4.84 | 6441 | 2 | 4740 | 1.3 |
| 11540 | 4.16 | 9840 | 3.75 | 8139 | 4.79 | 6438 | 2 | 4737 | 1.3 |
| 11537 | 4.16 | 9836 | 3.74 | 8135 | 4.68 | 6434 | 1.99 | 4733 | 1.3 |
| 11533 | 4.14 | 9832 | 3.72 | 8131 | 4.57 | 6430 | 1.99 | 4729 | 1.3 |
| 11529 | 4.09 | 9828 | 3.73 | 8127 | 4.54 | 6426 | 1.98 | 4725 | 1.3 |
| 11525 | 4.05 | 9824 | 3.75 | 8123 | 4.55 | 6422 | 1.98 | 4721 | 1.3 |
| 11521 | 3.99 | 9820 | 3.75 | 8119 | 4.56 | 6418 | 1.97 | 4717 | 1.31 |
| 11517 | 3.94 | 9816 | 3.73 | 8115 | 4.59 | 6414 | 1.97 | 4713 | 1.31 |
| 11513 | 3.98 | 9813 | 3.72 | 8112 | 4.58 | 6411 | 1.97 | 4710 | 1.31 |
| 11510 | 4.03 | 9809 | 3.73 | 8108 | 4.57 | 6407 | 1.96 | 4706 | 1.32 |
| 11506 | 4.03 | 9805 | 3.74 | 8104 | 4.57 | 6403 | 1.96 | 4702 | 1.33 |
| 11502 | 4.04 | 9801 | 3.75 | 8100 | 4.54 | 6399 | 1.96 | 4698 | 1.34 |
| 11498 | 4.1 | 9797 | 3.75 | 8096 | 4.49 | 6395 | 1.95 | 4694 | 1.35 |
| 11494 | 4.21 | 9793 | 3.73 | 8092 | 4.45 | 6391 | 1.95 | 4690 | 1.36 |
| 11490 | 4.33 | 9789 | 3.74 | 8088 | 4.43 | 6387 | 1.94 | 4686 | 1.37 |
| 11486 | 4.28 | 9786 | 3.75 | 8085 | 4.44 | 6384 | 1.94 | 4683 | 1.39 |
| 11483 | 4.42 | 9782 | 3.76 | 8081 | 4.44 | 6380 | 1.94 | 4679 | 1.41 |
| 11479 | 4.27 | 9778 | 3.75 | 8077 | 4.46 | 6376 | 1.93 | 4675 | 1.44 |
| 11475 | 4.21 | 9774 | 3.73 | 8073 | 4.45 | 6372 | 1.93 | 4671 | 1.46 |
| 11471 | 4.1 | 9770 | 3.71 | 8069 | 4.39 | 6368 | 1.92 | 4667 | 1.48 |

(continued)

| cm⁻¹ | Absorb. | cm⁻¹ | Absorb. | cm⁻¹ | Absorb. | cm⁻¹ | Absorb. | cm⁻¹ | Absorb. |
|---|---|---|---|---|---|---|---|---|---|
| 11467 | 4.12 | 9766 | 3.7 | 8065 | 4.35 | 6364 | 1.92 | 4663 | 1.5 |
| 11463 | 4.13 | 9762 | 3.7 | 8061 | 4.35 | 6360 | 1.92 | 4659 | 1.51 |
| 11459 | 4.07 | 9759 | 3.71 | 8058 | 4.37 | 6357 | 1.91 | 4656 | 1.53 |
| 11456 | 4.16 | 9755 | 3.72 | 8054 | 4.4 | 6353 | 1.91 | 4652 | 1.54 |
| 11452 | 4.15 | 9751 | 3.72 | 8050 | 4.44 | 6349 | 1.91 | 4648 | 1.56 |
| 11448 | 4.07 | 9747 | 3.73 | 8046 | 4.44 | 6345 | 1.9 | 4644 | 1.57 |
| 11444 | 4.09 | 9743 | 3.73 | 8042 | 4.38 | 6341 | 1.9 | 4640 | 1.59 |
| 11440 | 4.18 | 9739 | 3.73 | 8038 | 4.36 | 6337 | 1.9 | 4636 | 1.6 |
| 11436 | 4.28 | 9735 | 3.72 | 8034 | 4.39 | 6333 | 1.89 | 4632 | 1.61 |
| 11432 | 4.2 | 9732 | 3.72 | 8031 | 4.38 | 6330 | 1.89 | 4629 | 1.63 |
| 11429 | 4.1 | 9728 | 3.7 | 8027 | 4.35 | 6326 | 1.89 | 4625 | 1.64 |
| 11425 | 4.22 | 9724 | 3.69 | 8023 | 4.33 | 6322 | 1.88 | 4621 | 1.66 |
| 11421 | 4.38 | 9720 | 3.69 | 8019 | 4.3 | 6318 | 1.88 | 4617 | 1.67 |
| 11417 | 4.25 | 9716 | 3.7 | 8015 | 4.26 | 6314 | 1.88 | 4613 | 1.68 |
| 11413 | 4.15 | 9712 | 3.72 | 8011 | 4.25 | 6310 | 1.87 | 4609 | 1.69 |
| 11409 | 4.05 | 9708 | 3.74 | 8007 | 4.27 | 6306 | 1.87 | 4605 | 1.7 |
| 11405 | 4.01 | 9705 | 3.74 | 8004 | 4.29 | 6303 | 1.87 | 4602 | 1.7 |
| 11402 | 4.11 | 9701 | 3.73 | 8000 | 4.27 | 6299 | 1.86 | 4598 | 1.7 |
| 11398 | 4.2 | 9697 | 3.72 | 7996 | 4.25 | 6295 | 1.86 | 4594 | 1.7 |
| 11394 | 4.26 | 9693 | 3.72 | 7992 | 4.23 | 6291 | 1.86 | 4590 | 1.69 |
| 11390 | 4.3 | 9689 | 3.72 | 7988 | 4.25 | 6287 | 1.85 | 4586 | 1.68 |
| 11386 | 4.11 | 9685 | 3.72 | 7984 | 4.26 | 6283 | 1.85 | 4582 | 1.67 |
| 11382 | 4.01 | 9681 | 3.72 | 7980 | 4.26 | 6279 | 1.85 | 4578 | 1.67 |
| 11378 | 4.05 | 9678 | 3.71 | 7977 | 4.24 | 6276 | 1.84 | 4575 | 1.66 |
| 11375 | 4.04 | 9674 | 3.71 | 7973 | 4.2 | 6272 | 1.84 | 4571 | 1.65 |
| 11371 | 4.02 | 9670 | 3.72 | 7969 | 4.18 | 6268 | 1.84 | 4567 | 1.64 |
| 11367 | 3.97 | 9666 | 3.72 | 7965 | 4.18 | 6264 | 1.84 | 4563 | 1.64 |
| 11363 | 3.93 | 9662 | 3.71 | 7961 | 4.19 | 6260 | 1.83 | 4559 | 1.63 |
| 11359 | 3.93 | 9658 | 3.7 | 7957 | 4.19 | 6256 | 1.83 | 4555 | 1.63 |
| 11355 | 3.99 | 9654 | 3.7 | 7953 | 4.17 | 6252 | 1.83 | 4551 | 1.63 |
| 11351 | 4.09 | 9651 | 3.71 | 7950 | 4.15 | 6249 | 1.82 | 4548 | 1.64 |
| 11348 | 4.18 | 9647 | 3.71 | 7946 | 4.15 | 6245 | 1.82 | 4544 | 1.64 |
| 11344 | 4.19 | 9643 | 3.69 | 7942 | 4.16 | 6241 | 1.82 | 4540 | 1.65 |
| 11340 | 4.16 | 9639 | 3.67 | 7938 | 4.18 | 6237 | 1.82 | 4536 | 1.65 |
| 11336 | 4.16 | 9635 | 3.67 | 7934 | 4.18 | 6233 | 1.81 | 4532 | 1.66 |
| 11332 | 4.07 | 9631 | 3.7 | 7930 | 4.16 | 6229 | 1.81 | 4528 | 1.67 |
| 11328 | 3.96 | 9627 | 3.73 | 7926 | 4.13 | 6225 | 1.81 | 4524 | 1.68 |
| 11324 | 3.94 | 9624 | 3.72 | 7923 | 4.1 | 6222 | 1.81 | 4521 | 1.69 |

(continued)

| cm⁻¹ | Absorb. | cm⁻¹ | Absorb. | cm⁻¹ | Absorb. | cm⁻¹ | Absorb. | cm⁻¹ | Absorb. |
|---|---|---|---|---|---|---|---|---|---|
| 11321 | 3.95 | 9620 | 3.7 | 7919 | 4.07 | 6218 | 1.8 | 4517 | 1.7 |
| 11317 | 4.01 | 9616 | 3.68 | 7915 | 4.07 | 6214 | 1.8 | 4513 | 1.71 |
| 11313 | 4.07 | 9612 | 3.68 | 7911 | 4.09 | 6210 | 1.8 | 4509 | 1.73 |
| 11309 | 4 | 9608 | 3.68 | 7907 | 4.09 | 6206 | 1.8 | 4505 | 1.74 |
| 11305 | 3.89 | 9604 | 3.7 | 7903 | 4.07 | 6202 | 1.79 | 4501 | 1.76 |
| 11301 | 3.84 | 9600 | 3.72 | 7899 | 4.05 | 6198 | 1.79 | 4497 | 1.78 |
| 11297 | 3.88 | 9597 | 3.72 | 7896 | 4.05 | 6195 | 1.79 | 4494 | 1.8 |
| 11294 | 3.97 | 9593 | 3.71 | 7892 | 4.06 | 6191 | 1.79 | 4490 | 1.83 |
| 11290 | 4.03 | 9589 | 3.7 | 7888 | 4.05 | 6187 | 1.79 | 4486 | 1.85 |
| 11286 | 3.99 | 9585 | 3.71 | 7884 | 4.03 | 6183 | 1.79 | 4482 | 1.88 |
| 11282 | 3.89 | 9581 | 3.71 | 7880 | 4.02 | 6179 | 1.79 | 4478 | 1.92 |
| 11278 | 3.86 | 9577 | 3.7 | 7876 | 4.01 | 6175 | 1.78 | 4474 | 1.96 |
| 11274 | 3.87 | 9573 | 3.7 | 7872 | 3.99 | 6171 | 1.78 | 4470 | 2 |
| 11270 | 3.85 | 9570 | 3.72 | 7869 | 3.97 | 6168 | 1.78 | 4467 | 2.05 |
| 11267 | 3.83 | 9566 | 3.74 | 7865 | 3.97 | 6164 | 1.78 | 4463 | 2.11 |
| 11263 | 3.85 | 9562 | 3.72 | 7861 | 3.97 | 6160 | 1.78 | 4459 | 2.17 |
| 11259 | 3.85 | 9558 | 3.71 | 7857 | 3.96 | 6156 | 1.78 | 4455 | 2.25 |
| 11255 | 3.84 | 9554 | 3.72 | 7853 | 3.96 | 6152 | 1.78 | 4451 | 2.35 |
| 11251 | 3.82 | 9550 | 3.74 | 7849 | 3.96 | 6148 | 1.78 | 4447 | 2.46 |
| 11247 | 3.82 | 9546 | 3.74 | 7845 | 3.96 | 6144 | 1.79 | 4443 | 2.59 |
| 11243 | 3.82 | 9543 | 3.74 | 7842 | 3.95 | 6141 | 1.79 | 4440 | 2.76 |
| 11240 | 3.81 | 9539 | 3.74 | 7838 | 3.95 | 6137 | 1.79 | 4436 | 2.95 |
| 11236 | 3.78 | 9535 | 3.74 | 7834 | 3.94 | 6133 | 1.79 | 4432 | 3.19 |
| 11232 | 3.75 | 9531 | 3.76 | 7830 | 3.92 | 6129 | 1.79 | 4428 | 3.47 |
| 11228 | 3.75 | 9527 | 3.77 | 7826 | 3.91 | 6125 | 1.79 | 4424 | 3.79 |
| 11224 | 3.83 | 9523 | 3.77 | 7822 | 3.91 | 6121 | 1.79 | 4420 | 4.12 |
| 11220 | 3.95 | 9519 | 3.77 | 7818 | 3.9 | 6117 | 1.79 | 4416 | 4.45 |
| 11216 | 4 | 9516 | 3.73 | 7815 | 3.9 | 6114 | 1.79 | 4413 | 4.82 |
| 11213 | 3.93 | 9512 | 3.71 | 7811 | 3.89 | 6110 | 1.79 | 4409 | 5.13 |
| 11209 | 3.82 | 9508 | 3.71 | 7807 | 3.88 | 6106 | 1.79 | 4405 | 5.19 |
| 11205 | 3.79 | 9504 | 3.73 | 7803 | 3.88 | 6102 | 1.79 | 4401 | 5.15 |
| 11201 | 3.87 | 9500 | 3.76 | 7799 | 3.88 | 6098 | 1.79 | 4397 | 5.1 |
| 11197 | 3.95 | 9496 | 3.78 | 7795 | 3.88 | 6094 | 1.79 | 4393 | 5.05 |
| 11193 | 3.89 | 9492 | 3.78 | 7791 | 3.88 | 6090 | 1.79 | 4389 | 5.05 |
| 11189 | 3.79 | 9489 | 3.77 | 7788 | 3.87 | 6087 | 1.79 | 4386 | 5.09 |
| 11186 | 3.72 | 9485 | 3.75 | 7784 | 3.86 | 6083 | 1.79 | 4382 | 5.2 |
| 11182 | 3.74 | 9481 | 3.73 | 7780 | 3.84 | 6079 | 1.79 | 4378 | 5.34 |
| 11178 | 3.79 | 9477 | 3.73 | 7776 | 3.84 | 6075 | 1.79 | 4374 | 5.39 |

(continued)

| cm⁻¹ | Absorb. | cm⁻¹ | Absorb. | cm⁻¹ | Absorb. | cm⁻¹ | Absorb. | cm⁻¹ | Absorb. |
|---|---|---|---|---|---|---|---|---|---|
| 11174 | 3.81 | 9473 | 3.75 | 7772 | 3.83 | 6071 | 1.78 | 4370 | 5.2 |
| 11170 | 3.8 | 9469 | 3.77 | 7768 | 3.82 | 6067 | 1.78 | 4366 | 5.05 |
| 11166 | 3.8 | 9465 | 3.77 | 7764 | 3.81 | 6063 | 1.78 | 4362 | 5.07 |
| 11162 | 3.78 | 9462 | 3.76 | 7761 | 3.8 | 6060 | 1.78 | 4359 | 5.26 |
| 11159 | 3.74 | 9458 | 3.76 | 7757 | 3.79 | 6056 | 1.79 | 4355 | 5.26 |
| 11155 | 3.68 | 9454 | 3.76 | 7753 | 3.79 | 6052 | 1.79 | 4351 | 5.05 |
| 11151 | 3.63 | 9450 | 3.76 | 7749 | 3.79 | 6048 | 1.79 | 4347 | 4.98 |
| 11147 | 3.62 | 9446 | 3.77 | 7745 | 3.78 | 6044 | 1.79 | 4343 | 5.11 |
| 11143 | 3.64 | 9442 | 3.78 | 7741 | 3.78 | 6040 | 1.8 | 4339 | 5.29 |
| 11139 | 3.66 | 9438 | 3.78 | 7737 | 3.78 | 6036 | 1.8 | 4335 | 5.32 |
| 11135 | 3.64 | 9435 | 3.77 | 7734 | 3.77 | 6033 | 1.81 | 4332 | 5.36 |
| 11132 | 3.61 | 9431 | 3.76 | 7730 | 3.76 | 6029 | 1.82 | 4328 | 5.4 |
| 11128 | 3.6 | 9427 | 3.76 | 7726 | 3.75 | 6025 | 1.82 | 4324 | 5.28 |
| 11124 | 3.64 | 9423 | 3.77 | 7722 | 3.74 | 6021 | 1.83 | 4320 | 5.13 |
| 11120 | 3.65 | 9419 | 3.78 | 7718 | 3.73 | 6017 | 1.84 | 4316 | 4.99 |
| 11116 | 3.63 | 9415 | 3.78 | 7714 | 3.72 | 6013 | 1.86 | 4312 | 4.95 |
| 11112 | 3.6 | 9411 | 3.8 | 7710 | 3.72 | 6009 | 1.87 | 4308 | 4.95 |
| 11108 | 3.56 | 9408 | 3.81 | 7707 | 3.72 | 6006 | 1.88 | 4305 | 4.95 |
| 11105 | 3.54 | 9404 | 3.82 | 7703 | 3.7 | 6002 | 1.89 | 4301 | 4.95 |
| 11101 | 3.53 | 9400 | 3.83 | 7699 | 3.68 | 5998 | 1.91 | 4297 | 4.97 |
| 11097 | 3.52 | 9396 | 3.84 | 7695 | 3.66 | 5994 | 1.93 | 4293 | 5.01 |
| 11093 | 3.5 | 9392 | 3.83 | 7691 | 3.65 | 5990 | 1.94 | 4289 | 5.06 |
| 11089 | 3.5 | 9388 | 3.83 | 7687 | 3.65 | 5986 | 1.96 | 4285 | 5.1 |
| 11085 | 3.51 | 9384 | 3.84 | 7683 | 3.65 | 5982 | 1.98 | 4281 | 5.17 |
| 11081 | 3.52 | 9381 | 3.86 | 7680 | 3.65 | 5979 | 2 | 4278 | 5.37 |
| 11078 | 3.54 | 9377 | 3.87 | 7676 | 3.64 | 5975 | 2.02 | 4274 | 5.28 |
| 11074 | 3.54 | 9373 | 3.88 | 7672 | 3.63 | 5971 | 2.04 | 4270 | 5.36 |
| 11070 | 3.51 | 9369 | 3.89 | 7668 | 3.63 | 5967 | 2.07 | 4266 | 5.34 |
| 11066 | 3.5 | 9365 | 3.91 | 7664 | 3.63 | 5963 | 2.09 | 4262 | 5.21 |
| 11062 | 3.52 | 9361 | 3.92 | 7660 | 3.62 | 5959 | 2.11 | 4258 | 5.13 |
| 11058 | 3.53 | 9357 | 3.91 | 7656 | 3.61 | 5955 | 2.14 | 4254 | 5.15 |
| 11054 | 3.53 | 9354 | 3.9 | 7653 | 3.6 | 5952 | 2.17 | 4251 | 5.16 |
| 11051 | 3.52 | 9350 | 3.89 | 7649 | 3.6 | 5948 | 2.2 | 4247 | 5.23 |
| 11047 | 3.52 | 9346 | 3.91 | 7645 | 3.59 | 5944 | 2.24 | 4243 | 5.21 |
| 11043 | 3.51 | 9342 | 3.94 | 7641 | 3.59 | 5940 | 2.28 | 4239 | 5.19 |
| 11039 | 3.5 | 9338 | 3.99 | 7637 | 3.57 | 5936 | 2.34 | 4235 | 5.27 |
| 11035 | 3.5 | 9334 | 4.02 | 7633 | 3.56 | 5932 | 2.4 | 4231 | 5.24 |
| 11031 | 3.52 | 9330 | 4.02 | 7629 | 3.56 | 5928 | 2.48 | 4227 | 5.09 |

(continued)

| cm⁻¹ | Absorb. | cm⁻¹ | Absorb. | cm⁻¹ | Absorb. | cm⁻¹ | Absorb. | cm⁻¹ | Absorb. |
|---|---|---|---|---|---|---|---|---|---|
| 11027 | 3.53 | 9327 | 4.01 | 7626 | 3.55 | 5925 | 2.58 | 4224 | 5.08 |
| 11024 | 3.52 | 9323 | 4.01 | 7622 | 3.54 | 5921 | 2.68 | 4220 | 5.19 |
| 11020 | 3.53 | 9319 | 4.04 | 7618 | 3.54 | 5917 | 2.79 | 4216 | 5.39 |
| 11016 | 3.52 | 9315 | 4.06 | 7614 | 3.53 | 5913 | 2.89 | 4212 | 5.46 |
| 11012 | 3.5 | 9311 | 4.05 | 7610 | 3.53 | 5909 | 2.98 | 4208 | 5.34 |
| 11008 | 3.49 | 9307 | 4.06 | 7606 | 3.53 | 5905 | 3.03 | 4204 | 5.23 |
| 11004 | 3.5 | 9303 | 4.09 | 7602 | 3.52 | 5901 | 3.06 | 4200 | 5.14 |
| 11000 | 3.52 | 9300 | 4.1 | 7599 | 3.51 | 5898 | 3.07 | 4197 | 5.13 |
| 10997 | 3.54 | 9296 | 4.1 | 7595 | 3.5 | 5894 | 3.09 | 4193 | 5.1 |
| 10993 | 3.53 | 9292 | 4.11 | 7591 | 3.5 | 5890 | 3.12 | 4189 | 5.02 |
| 10989 | 3.52 | 9288 | 4.11 | 7587 | 3.49 | 5886 | 3.17 | 4185 | 5.03 |
| 10985 | 3.51 | 9284 | 4.11 | 7583 | 3.48 | 5882 | 3.25 | 4181 | 5.07 |
| 10981 | 3.51 | 9280 | 4.1 | 7579 | 3.48 | 5878 | 3.34 | 4177 | 5.09 |
| 10977 | 3.53 | 9276 | 4.1 | 7575 | 3.48 | 5874 | 3.44 | 4173 | 5.09 |
| 10973 | 3.55 | 9273 | 4.14 | 7572 | 3.47 | 5871 | 3.52 | 4170 | 5.14 |
| 10970 | 3.56 | 9269 | 4.18 | 7568 | 3.46 | 5867 | 3.57 | 4166 | 5.22 |
| 10966 | 3.55 | 9265 | 4.17 | 7564 | 3.46 | 5863 | 3.58 | 4162 | 5.26 |
| 10962 | 3.53 | 9261 | 4.17 | 7560 | 3.45 | 5859 | 3.57 | 4158 | 5.27 |
| 10958 | 3.53 | 9257 | 4.17 | 7556 | 3.44 | 5855 | 3.55 | 4154 | 5.33 |
| 10954 | 3.56 | 9253 | 4.17 | 7552 | 3.44 | 5851 | 3.53 | 4150 | 5.35 |
| 10950 | 3.6 | 9249 | 4.18 | 7548 | 3.43 | 5847 | 3.52 | 4146 | 5.29 |
| 10946 | 3.6 | 9246 | 4.2 | 7545 | 3.42 | 5844 | 3.53 | 4143 | 5.25 |
| 10943 | 3.58 | 9242 | 4.19 | 7541 | 3.41 | 5840 | 3.54 | 4139 | 5.27 |
| 10939 | 3.57 | 9238 | 4.19 | 7537 | 3.41 | 5836 | 3.58 | 4135 | 5.34 |
| 10935 | 3.59 | 9234 | 4.24 | 7533 | 3.4 | 5832 | 3.63 | 4131 | 5.21 |
| 10931 | 3.59 | 9230 | 4.38 | 7529 | 3.4 | 5828 | 3.69 | 4127 | 5.14 |
| 10927 | 3.59 | 9226 | 4.44 | 7525 | 3.4 | 5824 | 3.75 | 4123 | 5.07 |
| 10923 | 3.56 | 9222 | 4.3 | 7521 | 3.39 | 5820 | 3.82 | 4119 | 4.99 |
| 10919 | 3.53 | 9219 | 4.2 | 7518 | 3.38 | 5817 | 3.89 | 4116 | 4.96 |
| 10916 | 3.51 | 9215 | 4.21 | 7514 | 3.37 | 5813 | 3.96 | 4112 | 5.02 |
| 10912 | 3.51 | 9211 | 4.27 | 7510 | 3.36 | 5809 | 4.02 | 4108 | 5.19 |
| 10908 | 3.51 | 9207 | 4.33 | 7506 | 3.36 | 5805 | 4.07 | 4104 | 5.42 |
| 10904 | 3.52 | 9203 | 4.37 | 7502 | 3.35 | 5801 | 4.1 | 4100 | 5.49 |
| 10900 | 3.53 | 9199 | 4.35 | 7498 | 3.35 | 5797 | 4.11 | 4096 | 5.37 |
| 10896 | 3.54 | 9195 | 4.31 | 7494 | 3.34 | 5793 | 4.09 | 4092 | 5.28 |
| 10892 | 3.57 | 9192 | 4.3 | 7491 | 3.33 | 5790 | 4.07 | 4089 | 5.2 |
| 10889 | 3.59 | 9188 | 4.29 | 7487 | 3.33 | 5786 | 4.04 | 4085 | 5.09 |
| 10885 | 3.57 | 9184 | 4.3 | 7483 | 3.32 | 5782 | 3.99 | 4081 | 5.01 |

(continued)

| cm⁻¹ | Absorb. | cm⁻¹ | Absorb. | cm⁻¹ | Absorb. | cm⁻¹ | Absorb. | cm⁻¹ | Absorb. |
|---|---|---|---|---|---|---|---|---|---|
| 10881 | 3.56 | 9180 | 4.32 | 7479 | 3.32 | 5778 | 3.93 | 4077 | 4.97 |
| 10877 | 3.58 | 9176 | 4.32 | 7475 | 3.31 | 5774 | 3.86 | 4073 | 4.96 |
| 10873 | 3.61 | 9172 | 4.28 | 7471 | 3.31 | 5770 | 3.76 | 4069 | 4.99 |
| 10869 | 3.62 | 9168 | 4.22 | 7467 | 3.3 | 5766 | 3.66 | 4065 | 5.02 |
| 10865 | 3.62 | 9165 | 4.17 | 7464 | 3.29 | 5763 | 3.55 | 4062 | 5.01 |
| 10862 | 3.64 | 9161 | 4.16 | 7460 | 3.29 | 5759 | 3.46 | 4058 | 5.15 |
| 10858 | 3.64 | 9157 | 4.19 | 7456 | 3.28 | 5755 | 3.37 | 4054 | 5.36 |
| 10854 | 3.63 | 9153 | 4.23 | 7452 | 3.28 | 5751 | 3.28 | 4050 | 5.4 |
| 10850 | 3.62 | 9149 | 4.27 | 7448 | 3.27 | 5747 | 3.21 | 4046 | 5.31 |
| 10846 | 3.64 | 9145 | 4.34 | 7444 | 3.27 | 5743 | 3.14 | 4042 | 5.2 |
| 10842 | 3.68 | 9141 | 4.41 | 7440 | 3.26 | 5739 | 3.09 | 4038 | 5.14 |
| 10838 | 3.71 | 9138 | 4.43 | 7437 | 3.26 | 5736 | 3.05 | 4035 | 5.05 |
| 10835 | 3.7 | 9134 | 4.4 | 7433 | 3.25 | 5732 | 3.03 | 4031 | 4.95 |
| 10831 | 3.66 | 9130 | 4.39 | 7429 | 3.25 | 5728 | 3.02 | 4027 | 4.93 |
| 10827 | 3.64 | 9126 | 4.37 | 7425 | 3.24 | 5724 | 3.02 | 4023 | 5.05 |
| 10823 | 3.64 | 9122 | 4.29 | 7421 | 3.24 | 5720 | 3.03 | 4019 | 5.23 |
| 10819 | 3.65 | 9118 | 4.27 | 7417 | 3.23 | 5716 | 3.04 | 4015 | 5.32 |
| 10815 | 3.67 | 9114 | 4.31 | 7413 | 3.23 | 5712 | 3.05 | 4011 | 5.37 |
| 10811 | 3.68 | 9111 | 4.34 | 7410 | 3.22 | 5709 | 3.06 | 4008 | 5.14 |
| 10808 | 3.66 | 9107 | 4.34 | 7406 | 3.22 | 5705 | 3.08 | 4004 | 4.88 |
| 10804 | 3.63 | 9103 | 4.37 | 7402 | 3.21 | 5701 | 3.1 | 4000 | 4.81 |
| 10800 | 3.62 | 9099 | 4.43 | 7398 | 3.21 | 5697 | 3.12 | | |
| 10796 | 3.62 | 9095 | 4.44 | 7394 | 3.21 | 5693 | 3.15 | | |

**Claims**

1. A system for assigning a distillation temperature for a given distillation weight percentage to a fraction of an oil sample, without fractionation/distillation, wherein the oil sample is selected from naturally occurring hydrocarbons derived from crude oils, bitumens, heavy oils or shale oils, and is **characterized by** a density, the system comprising:

   a non-volatile memory device configured to store calculation modules and data, the data including near infrared, NIR, spectroscopy data indicative of absorbance values in a predetermined wavenumber range for the oil sample, as derived by near infrared spectroscopy analysis of the oil sample;
   a processor coupled to the memory;
   a first calculation module configured to calculate and assign a cumulative and normalized infrared absorbance for the given distillation weight percentage from the data indicative of absorbance values; and
   a second calculation module configured to calculate and assign a simulated distillation temperature of the fraction for the given distillation weight percentage from a multi variable degree 3 polynomial equation with predetermined constant coefficients developed using linear regression techniques, wherein the variables include the inverse of the wavenumber at known weight percent absorbance values and the inverse of the density of the oil sample.

2. A method for assigning a distillation temperature for a given distillation weight percentage to a fraction of an oil sample, without fractionation/distillation, wherein the oil sample is selected from naturally occurring hydrocarbons derived from crude oils, bitumens, heavy oils or shale oils, and is **characterized by** a density, the method comprising:

entering into a computer near infrared, NIR, spectroscopy data indicative of absorbance values in a predetermined wavenumber range for the oil sample, as derived by near infrared spectroscopy analysis of the crude oil sample;

calculating and assigning a cumulative and normalized infrared absorbance for the given distillation weight percentage from the data indicative of absorbance values; and

calculating and assigning a simulated distillation temperature of the fraction for the given distillation weight percentage from a multi variable degree 3 polynomial equation with predetermined constant coefficients developed using linear regression techniques, wherein the variables include the inverse of the wavenumber at known weight percentage absorbance values and the inverse of the density of the oil sample.

3. The system or method as in claim 1 or 2, wherein the oil sample is crude oil.

4. The system or method as in claim 1 or 2, wherein the oil sample is obtained from an oil well, stabilizer, extractor, or distillation tower.

5. The system or method as in claim 1 or 2, wherein plural distillation temperatures are assigned to obtain a set of simulated distillation data.

6. The system or method as in claim 5, wherein the given distillation weight percentage values are 0.5, 5, 10, 20, 30, 40, 50, 60, 70, 80 W%.

7. The system or method as in claim 1 or 2, wherein the predetermined wavenumber range is 4,000-12,821 cm$^{-1}$.

8. The system as in any of claims 1 or 3-7, further comprising a near infrared spectrometer that outputs the NIR spectroscopy data indicative of absorbance values.

9. The method as in any of claims 2 or 3-7, further comprising operating a near infrared spectrometer to obtain the NIR spectroscopy data indicative of absorbance values that is entered into the computer.

10. The system as in claim 1, wherein the second calculation module calculates and assigns the simulated distillation temperature with the equation:

$$T_{DT} = K_{SD} + X1_{SD}\left(\frac{1}{NIRWN}\right) + X2_{SD}\left(\frac{1}{DEN}\right) + X3_{SD}\left(\frac{1}{NIRWN^2}\right) + X4_{SD}\left(\frac{1}{DEN^2}\right) + X5_{SD}\left(\frac{1}{NIRWN*DEN}\right)$$

$$X6_{SD}\left(\frac{1}{NIRWN^3}\right) + X7_{SD}\left(\frac{1}{DEN^3}\right) + X8_{SD}\left(\frac{1}{NIRWN^2*DEN}\right) + X9_{SD}\left(\frac{1}{DEN^2*NIRWN}\right) \quad (1);$$

where:

DT is the distillation weight percentage, $K_{SD}$, $X1_{SD}$, $X2_{SD}$, $X3_{SD}$, $X4_{SD}$, $X5_{SD}$, $X6_{SD}$, $X7_{SD}$, $X8_{SD}$ and $X9_{SD}$ are constants, DEN is the density of the sample (kg/L), and NIRWN is the wavenumber at DT.

11. The method as in claim 2, wherein the simulated distillation temperature is calculated as $T_{DT}$ using the equation:

$$T_{DT} = K_{SD} + X1_{SD}\left(\frac{1}{NIRWN}\right) + X2_{SD}\left(\frac{1}{DEN}\right) + X3_{SD}\left(\frac{1}{NIRWN^2}\right) + X4_{SD}\left(\frac{1}{DEN^2}\right) + X5_{SD}\left(\frac{1}{NIRWN*DEN}\right)$$

$$X6_{SD}\left(\frac{1}{NIRWN^3}\right) + X7_{SD}\left(\frac{1}{DEN^3}\right) + X8_{SD}\left(\frac{1}{NIRWN^2*DEN}\right) + X9_{SD}\left(\frac{1}{DEN^2*NIRWN}\right) \quad (1);$$

where:

DT is the distillation weight percentage, $K_{SD}$, $X1_{SD}$, $X2_{SD}$, $X3_{SD}$, $X4_{SD}$, $X5_{SD}$, $X6_{SD}$, $X7_{SD}$, $X8_{SD}$ and $X9_{SD}$ are constants, DEN is the density of the sample (kg/L), and NIRWN is the wavenumber at DT.

**Patentansprüche**

1. System zum Zuordnen einer Destillationstemperatur für einen gegebenen Destillationsgewichtsprozentsatz zu einer Fraktion einer Ölprobe ohne Fraktionierung/Destillation, wobei die Ölprobe aus natürlich vorkommenden Kohlenwasserstoffen gewählt ist, die aus Rohölen, Bitumen, Schwerölen oder Schieferölen abgeleitet sind, und durch eine Dichte gekennzeichnet ist, wobei das System umfasst:

   eine nichtflüchtige Speichervorrichtung, die derart konfiguriert ist, Berechnungsmodule und Daten zu speichern, wobei die Daten Nahinfrarot-NIR-Spektroskopiedaten aufweisen, die Absorbanzwerte in einem vorbestimmten Wellenzahlbereich für die Ölprobe angeben, wie durch eine Nahinfrarotspektroskopieanalyse der Ölprobe abgeleitet ist;
   einen Prozessor, der mit dem Speicher gekoppelt ist;
   ein erstes Berechnungsmodul, das derart konfiguriert ist, eine kumulative und normalisierte Infrarotabsorbanz für den gegebenen Destillationsgewichtsprozentsatz von den Absorbanzwerte angebenden Daten zu berechnen und zuzuordnen; und
   ein zweites Berechnungsmodul, das derart konfiguriert ist, eine simulierte Destillationstemperatur der Fraktion für den gegebenen Destillationsgewichtsprozentsatz von einer Polynomgleichung 3. Grades mit mehreren Variablen mit vorbestimmten Konstantenkoeffizienten zu berechnen und zuzuordnen, die unter Verwendung von Linearregressionstechniken entwickelt sind, wobei die Variablen den Kehrwert der Wellenzahl bei bekannten Gewichtsprozentabsorbanzwerten und den Kehrwert der Dichte der Ölprobe aufweisen.

2. Verfahren zum Zuordnen einer Destillationstemperatur für einen gegebenen Destillationsgewichtsprozentsatz zu einer Fraktion einer Ölprobe ohne Fraktionierung/Destillation, wobei die Ölprobe aus natürlich vorkommenden Kohlenwasserstoffen gewählt ist, die aus Rohölen, Bitumen, Schwerölen oder Schieferölen abgeleitet sind, und durch eine Dichte gekennzeichnet ist, wobei das Verfahren umfasst, dass:

   Nahinfrarot-NIR-Spektroskopiedaten in einen Computer eingegeben werden, die Absorbanzwerte in einem vorbestimmten Wellenzahlbereich für die Ölprobe angeben, wie von einer Nahinfrarotspektroskopieanalyse der Rohölprobe abgeleitet ist;
   eine kumulative und normalisierte Infrarotabsorbanz für den gegebenen Destillationsgewichtsprozentsatz von den Absorbanzwerte angebenden Daten berechnet und zugeordnet wird; und
   eine simulierte Destillationstemperatur der Fraktion für den gegebenen Destillationsgewichtsprozentsatz von einer Polynomgleichung 3. Grades mit mehreren Variablen mit vorbestimmten Konstantenkoeffizienten berechnet und zugeordnet wird, die unter Verwendung von Linearregressionstechniken entwickelt sind, wobei die Variablen den Kehrwert der Wellenzahl bei bekannten Gewichtsprozentabsorbanzwerten und den Kehrwert der Dichte der Ölprobe aufweisen.

3. System oder Verfahren nach einem der Ansprüche 1 oder 2, wobei die Ölprobe Rohöl ist.

4. System oder Verfahren nach einem der Ansprüche 1 oder 2, wobei die Ölprobe aus einem Erdölbohrung, Stabilisator-, Extraktions- oder Destillationsturm erhalten ist.

5. System oder Verfahren nach einem der Ansprüche 1 oder 2, wobei mehrere Destillationstemperaturen zugeordnet sind, um einen Satz simulierter Destillationsdaten zu erhalten.

6. System oder Verfahren nach Anspruch 5, wobei die gegebenen Destillationsgewichtsprozentsätze 0,5, 5, 10, 20, 30, 40, 50, 60, 70, 80 Gew.-% sind.

7. System oder Verfahren nach einem der Ansprüche 1 oder 2, wobei der vorbestimmte Wellenzahlbereich 4.000 bis 12.821 cm$^{-1}$ ist.

8. System nach einem der Ansprüche 1 oder 3 - 7, ferner mit einem Nahinfrarotspektrometer, das NIR-Spektroskopiedaten ausgibt, die Absorbanzwerte angeben.

9. Verfahren nach einem der Ansprüche 2 oder 3 - 7, ferner umfassend, dass ein Nahinfrarotspektrometer betrieben wird, um die NIR-Spektroskopiedaten zu erhalten, die Absorbanzwerte angeben, die in den Computer eingegeben werden.

**10.** System nach Anspruch 1, wobei das zweite Berechnungsmodul die simulierte Destillationstemperatur mit der Gleichung berechnet und zuordnet:

$$T_{DT} = K_{SD} + X1_{SD}\left(\frac{1}{NIRWN}\right) + X2_{SD}\left(\frac{1}{DEN}\right) + X3_{SD}\left(\frac{1}{NIRWN^2}\right) + X4_{SD}\left(\frac{1}{DEN^2}\right) + X5_{SD}\left(\frac{1}{NIRWN*DEN}\right)$$
$$X6_{SD}\left(\frac{1}{NIRWN^3}\right) + X7_{SD}\left(\frac{1}{DEN^3}\right) + X8_{SD}\left(\frac{1}{NIRWN^2*DEN}\right) + X9_{SD}\left(\frac{1}{DEN^2*NIRWN}\right) \quad (1);$$

wobei:
DT der Destillationsgewichtsprozentsatz ist, KSD, $X1_{SD}$, $X2_{SD}$, $X3_{SD}$, $X4_{SD}$, $X5_{SD}$, $X6_{SD}$, $X7_{SD}$, $X8_{SD}$ und $X9_{SD}$ Konstanten sind, DEN die Dichte der Probe (kg/l) ist, und NIRWN die Wellenzahl bei DT ist.

**11.** Verfahren nach Anspruch 2, wobei die simulierte Destillationstemperatur als $T_{DT}$ unter Verwendung der Gleichung berechnet ist:

$$T_{DT} = K_{SD} + X1_{SD}\left(\frac{1}{NIRWN}\right) + X2_{SD}\left(\frac{1}{DEN}\right) + X3_{SD}\left(\frac{1}{NIRWN^2}\right) + X4_{SD}\left(\frac{1}{DEN^2}\right) + X5_{SD}\left(\frac{1}{NIRWN*DEN}\right)$$
$$X6_{SD}\left(\frac{1}{NIRWN^3}\right) + X7_{SD}\left(\frac{1}{DEN^3}\right) + X8_{SD}\left(\frac{1}{NIRWN^2*DEN}\right) + X9_{SD}\left(\frac{1}{DEN^2*NIRWN}\right) \quad (1);$$

wobei:
DT der Destillationsgewichtsprozentsatz ist, KSD, $X1_{SD}$, $X2_{SD}$, $X3_{SD}$, $X4_{SD}$, $X5_{SD}$, $X6_{SD}$, $X7_{SD}$, $X8_{SD}$ und $X9_{SD}$ Konstanten sind, DEN die Dichte der Probe (kg/l) ist, und NIRWN die Wellenzahl bei DT ist.

## Revendications

**1.** Système d'attribution d'une température de distillation pour un pourcentage en poids donné de distillation à une fraction d'un échantillon d'huile, sans fractionnement/distillation, dans lequel l'échantillon d'huile est sélectionné parmi des hydrocarbures naturels provenant des pétroles bruts, des bitumes, des huiles lourdes ou des huiles de schistes, et est **caractérisé par** une densité, le système comprenant :

un dispositif à mémoire non volatile configuré pour mémoriser des modules de calcul et des données, les données comportant des données de spectroscopie proche infrarouge, NIRS, indicatives de valeurs d'absorbance dans une plage de nombres d'ondes prédéterminée pour l'échantillon d'huile, dérivées d'une analyse de spectroscopie proche infrarouge de l'échantillon d'huile ;
un processeur couplé à la mémoire ;
un premier module de calcul configuré pour calculer et attribuer une absorbance infrarouge cumulative et normalisée pour le pourcentage en poids donné de distillation à partir des données indicatives de valeurs d'absorbance ; et
un deuxième module de calcul configuré pour calculer et attribuer une température de distillation simulée de la fraction pour le pourcentage en poids donné de distillation à partir d'une équation polynomiale de degré 3 multivariable avec des coefficients constants prédéterminés développés en utilisant des techniques de régression linéaire, dans lequel les variables comprennent l'inverse du nombre d'ondes en valeurs d'absorbance de pourcentage en poids connues et l'inverse de la densité de l'échantillon de l'huile.

**2.** Procédé d'attribution d'une température de distillation pour un pourcentage en poids donné de distillation à une fraction d'un échantillon d'huile, sans fractionnement/distillation, dans lequel l'échantillon d'huile est sélectionné parmi des hydrocarbures naturels provenant des pétroles bruts, des bitumes, des huiles lourdes ou des huiles de schistes, et est **caractérisé par** une densité, le procédé comprenant :

insérer dans un ordinateur des données de spectroscopie proche infrarouge, NIRS, indicatives de valeurs d'absorbance dans une plage de nombres d'ondes prédéterminée pour l'échantillon d'huile, dérivées d'une analyse de spectroscopie proche infrarouge de l'échantillon de pétrole brut;
calculer et attribuer une absorbance infrarouge cumulative et normalisée pour le pourcentage en poids donné de distillation à partir des données indicatives de valeurs d'absorbance ; et

calculer et attribuer une température de distillation simulée de la fraction pour le pourcentage en poids donné de distillation à partir d'une équation polynomiale de degré 3 multivariable avec des coefficients constants prédéterminés développés en utilisant des techniques de régression linéaire, dans lequel les variables comprennent l'inverse du nombre d'ondes en valeurs d'absorbance de pourcentage en poids connues et l'inverse de la densité de l'échantillon de l'huile.

3. Système ou procédé selon la revendication 1 ou 2, dans lequel l'échantillon d'huile est du pétrole brut.

4. Système ou procédé selon la revendication 1 ou 2, dans lequel l'échantillon d'huile est obtenu d'un puit de pétrole, un stabilisateur, un extracteur ou une tour de distillation.

5. Système ou procédé selon la revendication 1 ou 2, dans lequel plusieurs températures de distillation sont attribuées afin d'obtenir un ensemble de données de distillation simulées.

6. Système ou procédé selon la revendication 5, dans lequel les valeurs de pourcentage en poids donné de distillation sont 0,5, 5, 10, 20, 30, 40, 50, 60, 70, 80 % en poids.

7. Système ou procédé selon la revendication 1 ou 2, dans lequel la plage de nombres d'ondes prédéterminée est 4,000-12,821 cm$^{-1}$.

8. Système selon l'une quelconque des revendications 1 ou 3 à 7, comprenant en outre un spectromètre proche infrarouge qui émet les données de spectroscopie NIRS indicatives de valeurs d'absorbance.

9. Procédé selon l'une quelconque des revendications 2 ou 3 à 7, comprenant en outre l'exploitation d'un spectromètre proche infrarouge pour obtenir les données de spectroscopie NIRS indicatives de valeurs d'absorbance qui sont insérées dans l'ordinateur.

10. Système selon la revendication 1, dans lequel le deuxième module de calcul calcule et attribue la température de distillation simulée avec l'équation :

$$T_{DT} = K_{SD} + X1_{SD}\left(\frac{1}{NIRWN}\right) + X2_{SD}\left(\frac{1}{DEN}\right) + X3_{SD}\left(\frac{1}{NIRWN^2}\right) + X4_{SD}\left(\frac{1}{DEN^2}\right) + X5_{SD}\left(\frac{1}{NIRWN * DEN}\right)$$
$$X6_{SD}\left(\frac{1}{NIRWN^3}\right) + X7_{SD}\left(\frac{1}{DEN^3}\right) + X8_{SD}\left(\frac{1}{NIRWN^2 * DEN}\right) + X9_{SD}\left(\frac{1}{DEN^2 * NIRWN}\right) \quad (1);$$

où :
DT est le pourcentage en poids de distillation, $K_{SD}$, $X1_{SD}$, $X2_{SD}$, $X3_{SD}$, $X4_{SD}$, $X5_{SD}$, $X6_{SD}$, $X7_{SD}$, $X8_{SD}$ et $X9_{SD}$ sont des constants, DEN est la densité de l'échantillon (kg/L), et NIRWN est le nombre d'ondes à DT.

11. Procédé selon la revendication 2, dans lequel la température de distillation simulée est calculée comme $T_{DT}$ en utilisant l'équation :

$$T_{DT} = K_{SD} + X1_{SD}\left(\frac{1}{NIRWN}\right) + X2_{SD}\left(\frac{1}{DEN}\right) + X3_{SD}\left(\frac{1}{NIRWN^2}\right) + X4_{SD}\left(\frac{1}{DEN^2}\right) + X5_{SD}\left(\frac{1}{NIRWN * DEN}\right)$$
$$X6_{SD}\left(\frac{1}{NIRWN^3}\right) + X7_{SD}\left(\frac{1}{DEN^3}\right) + X8_{SD}\left(\frac{1}{NIRWN^2 * DEN}\right) + X9_{SD}\left(\frac{1}{DEN^2 * NIRWN}\right) \quad (1);$$

où :
DT est le pourcentage en poids de distillation, $K_{SD}$, $X1_{SD}$, $X2_{SD}$, $X3_{SD}$, $X4_{SD}$, $X5_{SD}$, $X6_{SD}$, $X7_{SD}$, $X8_{SD}$ et $X9_{SD}$ sont des constants, DEN est la densité de l'échantillon (kg/L), et NIRWN est le nombre d'ondes à DT.

*FIG. 1*

```
                    ┌─────────────┐
                    │    Start    │
                    └──────┬──────┘
                           │
                           ▼
    210 ──┐  ┌──────────────────────────┐
          │  │      Obtain density      │
          │  └────────────┬─────────────┘
                          │
                          ▼
    220 ──┐  ┌──────────────────────────┐
          │  │   Perform near infrared  │
          │  │       spectroscopy       │
          │  └────────────┬─────────────┘
                          │
                          ▼
    230 ──┐  ┌──────────────────────────┐
          │  │  Enter density & spectra │
          │  │   data into computer     │
          │  └────────────┬─────────────┘
                          │
                          ▼
    240 ──┐  ┌──────────────────────────┐
          │  │   Calculate simulated    │
          │  │    distillation data     │
          │  └────────────┬─────────────┘
                          │
                          ▼
                    ┌─────────────┐
                    │     End     │
                    └─────────────┘
```

*FIG. 2*

*FIG. 3*

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 62099704
- US 5710578 A

- US 211928

**Non-patent literature cited in the description**

- **F.S. FALLA et al.** Characterization of crude petroleum by NIR. *Journal of Petroleum Science and Engineering,* 2006, vol. 51